(19) Europäisches Patentamt / European Patent Office / Office européen des brevets

(11) **EP 4 617 960 A2**

(12) **EUROPEAN PATENT APPLICATION**

(43) Date of publication:
**17.09.2025 Bulletin 2025/38**

(21) Application number: **25192289.4**

(22) Date of filing: **27.03.2020**

(51) International Patent Classification (IPC):
***G06N 20/00*** (2019.01)

(52) Cooperative Patent Classification (CPC):
**G06N 3/126; G05B 17/02; G06N 3/045; G06N 3/08; G06N 5/01; G06N 20/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2019 US 201962826910 P**

(62) Document number(s) of the earlier application(s) in accordance with Art. 76 EPC:
**20782758.5 / 3 948 276**

(71) Applicant: **Amgen Inc.**
**Thousand Oaks, CA 91320 (US)**

(72) Inventors:
• **JOHNSON, William Leigh**
**California, 91320-1799 (US)**
• **CÁRCAMO BEHRENS, Martin Rodrigo**
**California, 91320-1799 (US)**

(74) Representative: **Korenberg, Alexander Tal et al**
**Kilburn & Strode LLP**
**Lacon London**
**84 Theobalds Road**
**London WC1X 8NL (GB)**

Remarks:
This application was filed on 28-07-2025 as a divisional application to the application mentioned under INID code 62.

(54) **PREDICTING CELL CULTURE PERFORMANCE IN BIOREACTORS**

(57) A computational method of modeling a bioreactor combines mechanistic models of kinetics of metabolic fluxes and flux balance analysis to predict cell culture performance. The mechanistic models include effects of process variables descriptive of extracellular environment, *e.g.*, temperature, acidity, osmolarity, and/or metabolite concentrations. The method constrains flux rates based on the mechanistic models and computes the flux rates in view of suitable metabolic objectives. The method simulates time evolution of process variables of the bioreactor based on user input and computes performance metrics to display to the user, to control a bioreactor, and/or to train an artificial intelligence model of a bioreactor.

EP 4 617 960 A2

## Description

### FIELD OF DISCLOSURE

This disclosure generally relates to methods of *in silico* modeling of bioreactors and the associated biological processes for the purpose of improving bioprocess design and/or operation.

### BACKGROUND

Biotechnology uses living cells to produce valuable biochemical compounds. Different cells can be engineered to produce a wide variety of target biochemical products for medicinal, agricultural, or other uses. Practitioners construct and use bioreactors for growing cell cultures that produce target products along with other metabolic byproducts. Maximizing the target product volume and quality for a given cost of production is one of the main goals of bioreactor design.

A bioreactor may be configured and controlled to operate in a variety of modes and with different goals. The first goal of the process may be to grow a sufficient amount of cell culture without using more than the necessary amount of substrate nutrients. The type of cellular biomass grown in the bioreactor may depend on an application, with some popular bio-cultures, such as Chinese hamster ovary (CHO) cells, being used for a number of different applications. In one mode of operation of a bioreactor, called a batch mode, growth of a bio-culture may take place without any liquid flowing into or out of the reactor, particularly if the bioreactor control system can maintain good conditions for growth. Good conditions for growth may require appropriate temperature, acidity, and concentrations of substrate metabolites. As the growing biomass consumes substrate metabolites, an input flow may help replenish the metabolites needed for growth, forming a fed-batch configuration of a bioreactor. Metabolic byproducts produced during growth may compromise the optimality of growth conditions. Adding input and output flow streams, forming a continuous bioreactor configuration, may alleviate the negative effects of metabolic byproducts as well as offer more control over the duration of the cell growth stage. A filter may be added to the output stream to prevent removal of cellular biomass, forming a perfusion configuration of the bioreactor.

Once a sufficient amount of biomass is produced in the bioreactor, the next stage of operation may prioritize generation and collection of a target metabolic product. Such target products may include therapeutic antibodies or other biologics. Inducing cells to shift metabolic energy from growing the biomass to production of the target product often involves introducing a stress factor. The stress may comprise a reduction in optimal host temperature, causing the cells to expend more energy on non-growth metabolism that can, in turn, result in increased target production.

Optimizing all the stages of the bioreactor operations poses a significant challenge. The many decisions may include when to transition from growth to production, when to begin harvesting, when and in what quantities to supply and remove metabolites, how to control the bioreactor environment. Often, many expensive and time consuming *in vivo* experiments are performed to select operating conditions. Alternative methods for optimizing and controlling bioreactor operations would bring considerable benefits to the industry and the public it serves.

### SUMMARY

A computational method of modeling a bioreactor is used to predict cell culture performance, to improve and/or optimize bioreactor design and/or operation. In one aspect, the method comprises receiving a plurality of current values of process variables, the process variables describing virtual contents of the modeled bioreactor, and the virtual contents including virtual cellular biomass in a virtual extracellular solution. The method also comprises computing, by processing hardware of a computing system, new values of the process variables during a simulated time period. The method computes the new values at least in part by: generating a plurality of constraints on flux rates of metabolic fluxes describing the virtual cellular biomass by modeling one or more effects of at least some of the current values of the process variables on metabolic reaction kinetics; computing the flux rates of the metabolic fluxes by performing flux balance analysis subject to a metabolic objective and the generated plurality of the constraints on the flux rates; computing rates of change of at least some of the process variables based at least in part on the computed flux rates; and updating one or more of the current values of the process variables at least in part by integrating one or more of the computed rates of change for a virtual time step within the simulated time period. The method also comprises computing, by the processing hardware, a metric of the computationally modeled bioreactor based on the computed new values of the process variables, and generating, by the processing hardware, and based on the metric of the modeled bioreactor one or more of: i) information displayed to a user via a user interface, ii) a control setting for a real-world bioreactor, or iii) a training set for an artificial intelligence model of a bioreactor. For any method described herein, the method may optionally further comprise culturing mammalian cells in a bioreactor using one or more parameters identified in the modeled bioreactor. By way of example, the mammalian cells may encode a therapeutic protein, and may produce the therapeutic protein when cultured in the bioreactor. Examples of mammalian cells include CHO cells and BHK cells. For conciseness a "bioreactor" may also be referred to herein as a "reactor," and unless explicitly stated otherwise, these terms will be understood to be interchangeable herein.

In another aspect, non-transitory computer-readable medium stores instructions for computationally modeling a bioreactor, wherein the instructions, when executed by one or more processors, cause the one or more processors to receive a plurality of current values of process variables, the process variables describing virtual contents of the modeled bioreactor, and the virtual contents including virtual cellular biomass in a virtual extracellular solution. The instructions also cause the one or more processors to compute, by processing hardware of a computing system, new values of the process variables during a simulated time period. The one or more processors compute the new values at least in part by: generating a plurality of constraints on flux rates of metabolic fluxes describing the virtual cellular biomass by modeling one or more effects of at least some of the current values of the process variables on metabolic reaction kinetics; computing the flux rates of the metabolic fluxes by performing flux balance analysis subject to a metabolic objective and the generated plurality of the constraints on the flux rates; computing rates of change of at least some of the process variables based at least in part on the computed flux rates; and updating one or more of the current values of the process variables at least in part by integrating one or more of the computed rates of change for a virtual time step within the simulated time period. The instructions also cause the one or more processors to compute, by the processing hardware, a metric of the modeled bioreactor based on the computed new values of the process variables, and generate, by the processing hardware, and based on the metric of the modeled bioreactor one or more of: i) information displayed to a user via a user interface, ii) a control setting for a real-world bioreactor, or iii) a training set for an artificial intelligence model of a bioreactor.

## BRIEF DESCRIPTION OF THE DRAWINGS

Figure 1 illustrates an exemplary process for computationally modeling a bioreactor, and a corresponding computational model.

Figure 2 is a graph depicting an exemplary effect of a temperature shift on a stress variable in an implementation of the modeling process of Figure 1.

Figure 3 illustrates an exemplary computer system that may implement the modeling process of Figure 1.

Figures 4A and 4B illustrate panels of an exemplary user interface for receiving user inputs.

Figure 5 illustrates an exemplary user interface for displaying outputs to a user.

Figure 6 illustrates an exemplary bioreactor control system.

Figure 7 is a flow chart depicting an exemplary method of computationally modeling a bioreactor.

Figure 8 illustrates a comparison between a bioreactor model and experimental observations.

Figures 9A and 9B illustrate a relationship between a metabolic model of an inhomogeneous bioreactor modeled with homogeneous portions and a velocity field that may be computed using computational fluid dynamic (CFD).

## DETAILED DESCRIPTION

Much of the description herein focuses on a metabolic (biological and biochemical) model of a bioreactor configured, for example, to produce a certain biological product. Though the description mostly treats a bioreactor as a homogeneous mixture of extracellular solution and a cellular biomass, the metabolic model may readily be adapted to account for spatial inhomogeneity in a bioreactor. To that end, the metabolic model may be integrated *in silico* with a computational model of transport physics. Thus, certain portions of this description describe model integration, demonstrating the general applicability of the metabolic model to bioreactors that need not be well-mixed.

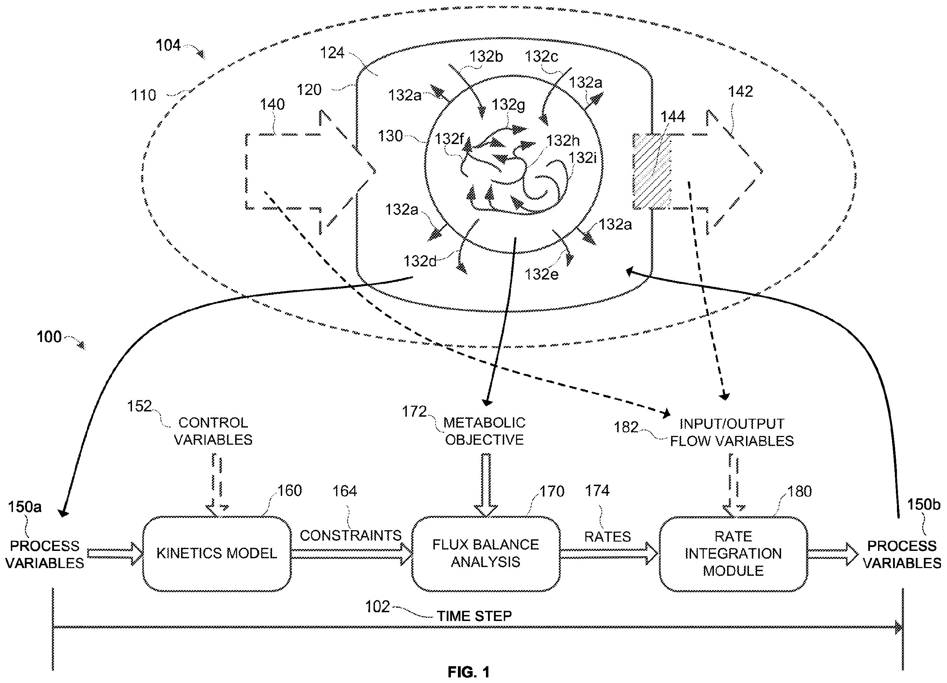

Figure 1 illustrates an exemplary process 100 for computationally modeling a bioreactor in a series of time steps, including a depicted time step 102, with a corresponding computational model 104 represented in a virtual space 110. It is understood that the time step 102 may be repeated any suitable number of times during the modeling process 100. Virtual contents of a modeled bioreactor 120 may include a virtual extracellular solution 124 and virtual cellular biomass 130 disposed within the virtual extracellular solution 124. For convenience, the modeled bioreactor 120 may be referred to herein as a "bioreactor," the virtual extracellular solution 124 may be referred to herein as an "extracellular solution" (or, simply, "solution"), and the virtual cellular biomass 130 may be referred to herein as a "cellular biomass" (or, simply, "biomass"). A collection of fluxes 132a-i may at least in part represent the state of the biomass 130. A virtual input stream 140 and a virtual output stream 142 may, respectively, replenish and deplete the virtual contents of the modeled bioreactor 120. A virtual filter 144 may selectively facilitate, allow, restrict or prevent the flows of some contents out of the modeled bioreactor 120. For example, to model a bioreactor in perfusion mode of operation, the filter 144 may allow the flow of the extracellular solution 124, while restricting or preventing the flow of the cellular biomass 130. In contrast, excluding or zeroing the virtual input and output streams 140, 142 models a bioreactor in batch mode of operation. Generally, changing the flow and/or compositions of virtual input and output streams 140, 142 may allow modeling batch, fed-batch, continuous, and/or perfusion configurations or modes of operation of the bioreactor 120.

In some implementations, the modeling process 100 may be configured to model spatial heterogeneity of a bioreactor. The spatial heterogeneity may refer to, for example, spatial variations in composition of extracellular solution, concentration or composition of cellular biomass, or in other process parameters (e.g., temperature) at different locations within a bioreactor. In a well-mixed bioreactor, such differences may be small and a homogeneous model may simulate a well-mixed bioreactor with sufficient accuracy. In modeling less well-mixed bioreactors, on the other hand, computationally accounting for the spatial inhomogeneity can substantially improve accuracy of the model. To that end, the metabolic model described here can be coupled with physical models of mass and heat transport within a reactor. In some implementations, computational fluid dynamics (CFD) models may cooperate with the metabolic model as discussed herein. Generally, modeling the inhomogeneity may be performed in view of a trade-off between accuracy and computational complexity.

To model spatial inhomogeneity using the modeling process 100, the modeled/virtual bioreactor 120 may model a portion of an inhomogeneous bioreactor. Thus, in the context of a spatially inhomogeneous bioreactor model, the bioreactor 120 may be thought of as a portion of an inhomogeneous bioreactor. That is, the modeled inhomogeneous bioreactor may be divided into portions in any one of various suitable ways, depending, for example, on the degree of inhomogeneity and the coupled physical model. In some implementations, at least 2, 5, 10, 20 or another suitable number of portions (including ranges between any two of the listed values) of varied dimensions may represent different regions of a bioreactor (e.g., inlets and outlets, central portion, etc.). In some implementations, the bioreactor volume may be divided into at least 100, 1000, 10000, 100000, 1000000 or any other suitable number of portions (including ranges between any two of the listed values) represented by finite volume elements, that may also, for example, serve as finite volume elements of a CFD model.

The modeled portion represented by the bioreactor 120 may be assumed to be substantially homogeneous. Consequently, the extracellular solution 124 may model a portion of the extracellular solution corresponding to the modeled portion of the inhomogeneous reactor, and the biomass 130 may model a corresponding portion of biomass distributed throughout the inhomogeneous bioreactor. The input stream 140 may model mass transport (i.e., flow of extracellular solution and biomass) into the portion (modeled by bioreactor 120) of the inhomogeneous bioreactor from one or more neighboring portions of the inhomogeneous bioreactor. Analogously, the output stream 142 may model mass transport (i.e., flow of extracellular solution and biomass) out of the portion (represented by bioreactor 120) of the inhomogeneous bioreactor into neighboring portions of the inhomogeneous bioreactor. In this manner, a collection of homogeneous bioreactor models (each representing a portion of an inhomogeneous bioreactor) may be linked to model the inhomogeneous bioreactor. Input and output streams that replenish and deplete the inhomogeneous bioreactor may be added to the input and output streams 140, 142 of a portion of the inhomogeneous bioreactor with inputs from or outputs to the outside of the inhomogeneous bioreactor. In such a case, at least portions of the input and output streams 140, 142 can be thought to account for input and output boundary conditions of the inhomogeneous bioreactor model assembled from homogeneous bioreactor models.

A computational (e.g., CFD) model may be configured to compute input and output streams (e.g., streams 140,142) of each portion (e.g., bioreactor 120) of the inhomogeneous bioreactor model. In some implementations, each portion of the inhomogeneous bioreactor modeled by the process 100 may correspond to a finite volume element of the CFD model, as discussed above. Generally, however, spatial discretization for the CFD model need not be the same as spatial discretization into reactor portions for applying the modeling process 100. For example, the CFD model may compute a velocity field within the modeled inhomogeneous bioreactor at one spatial resolution. The velocity field may be combined with the composition of the bioreactor computed by the metabolic model at a different spatial resolution to update the input and output streams 140, 142 for each of the bioreactor portions represented by the metabolic model.

The computation of a velocity field may depend on the physical configuration (e.g., the mixing mechanisms) of the bioreactor. In some bioreactors, natural convection may be the dominant mixing mechanism. That is, temperature gradients due to metabolic processes or reactor temperature control may drive convection. In other implementations, injected fluids, such as liquid substrate or gas, may drive convection. Additionally or alternatively, impellers or other suitable mixing mechanisms may force the mixing processes. Computational or empirical models may be used to compute a velocity field at suitable time scales. For example, once computed, a velocity field may be assumed to be substantially constant until there is a substantive change in a physical property (e.g., viscosity change due to biomass growth) of bioreactor contents or in a manner of driving the mixing process (e.g., change in input or output flow rates imposed by the control of the bioreactor).

Temporal resolutions of the metabolic model and the physical model, like the spatial resolutions, need not be the same, at least in view of the above discussion. That is, the time step 102 of the modeling process 100, describes the metabolic process model. Aspects of integrating the modeling process 100, applied to portions of an inhomogeneous bioreactor, with the modeling of mixing (e.g., by CFD) or other physical processes within the inhomogeneous reactor are referenced herein, as needed.

The modeling process 100 and the computational model 104, implemented in software that is running on hardware of a computing system, may simulate operation of a virtual bioreactor 120 over a simulated time period composed of the series of time steps 102. The modeling process 100 comprises receiving (from a user, or from a previous time step 102) current values 150a of process variables at the beginning of a time step 102 and, at the end of the time step 102, generating updated values 150b of at least some of those process variables through a series of computational steps. In contexts where the precise timing relative to any given time step 102 is not at issue, the current values 150a and updated values 150b may be collectively referred to herein simply as "values" of process variables. Process variables may be a set of variables describing the computational model 104 of the bioreactor. In some implementations, the process variables may reflect values of control variables 152, process conditions, experimental conditions, media composition, bioreactor parameters, model constants, and/or a variety of other terms describing parameters of the computational model 104. Process variables may include variables descriptive or indicative of a variety of physical, chemical and biochemical properties of the extracellular solution 124, such as volume, temperature, acidity or pH, osmolarity, osmolality, mass, density as well as chemical and biochemical compositions of the solution. Additionally, process variables may indicate the amount or concentration of the cellular biomass 130 disposed in the extracellular solution 124 within the modeled bioreactor 120. Additionally or alternatively, process variables 150 may indicate concentrations or absolute amounts of a variety of inorganic or organic metabolites disposed in the extracellular solution 124 or within the biomass 130. The dissolved metabolites, with extracellular or intracellular concentrations indicated by the process variables 150, may include gasses, salts, ions, metals, minerals, sugars, amino acids, fatty acids, and/or other organic acids and/or their conjugate bases. More specifically, the dissolved metabolites may include oxygen, carbon dioxide, ammonia, glucose, sucrose, lactose, lactate, glutamine, glutamate, alanine, asparagine, aspartate and/or a variety of other chemicals or biochemicals. Process variables may also indicate concentrations of one or more target metabolic products (or, simply, target products), that may include antibodies, peptides, therapeutic proteins or other biologics. Process variables may also indicate concentrations of one or more of a variety of metabolic byproduct (or, simply, byproducts), that, along with target products, may be generated by virtual cellular biomass 120. Target products and byproducts may collectively be called metabolic products (or, simply, products).

To determine the updated values 150b of the process variables from the current values 150a of the process variables, the modeling process 100 may compute the velocities or rates of at least some of the fluxes 132a-i associated with the cellular biomass 130. The fluxes 132a-i may correspond to the growth 132a of the cellular biomass 130, uptake of substrate metabolites 132b, c, removal or secretion of metabolic target products and byproducts 132 d, e, and/or fluxes representing a variety of intracellular metabolic pathway fluxes132f-i. The intracellular metabolic pathway fluxes 132f-i may represent metabolic conversions of a number of metabolites into a number of other metabolites, as indicated in Figure 1 by the topologies of the flux representations. For example, flux 132f may represent a reaction or metabolic pathway where two metabolites combine to produce another metabolite, flux 132g may represent a reaction or metabolic pathway where one metabolite produces two other metabolites, flux 132h may represent a reaction or metabolic pathway where two metabolites combine to produce two other metabolites, and flux 132i may represent a reaction or metabolic pathway where two metabolites combine to produce three metabolites. The growth flux 132a may in some implementations be modeled as an output of another flux that additionally represents an intracellular metabolic process. Collectively, the fluxes 132a-i may represent a metabolic network for modeling a given type of cells making the cellular biomass 130. The metabolic network for modeled bacterial cells, such as *Escherichia coli* (*E. coli*), may be different from the metabolic network for modeled mammalian reproductive cells, such as CHO cells. The fluxes 132a-i in Figure 1 are for general illustration, and a metabolic network may contain fluxes that contain from zero to more than a dozen inputs and/or outputs. Furthermore, a metabolic network may comprise 5, 20, 100, 500, 2500 or any other suitable number of metabolic pathways.

The intracellular metabolites disposed in the cellular biomass 130 modeled by the fluxes 132a-i of the metabolic network may include some or all of the metabolites modeled in the extracellular solution: oxygen, carbon dioxide, ammonia, other gasses, salts, ions, metals, minerals, glucose, sucrose, lactose, lactate, glutamine, glutamate, glycine, alanine, asparagine, aspartate and/or a variety of other chemicals or biochemicals. The intracellular metabolites participating in the fluxes 132a-i may also include a variety of enzymes, proteins, amino acids, and/or ADP (adenosine diphosphate) and ATP (adenosine triphosphate) molecules involved in energy transfer. Variables indicative of intracellular concentrations of metabolites may be included in the process variables.

Each metabolic pathway flux may be quantified by a flux rate which characterizes the rate of conversion of the metabolites associated with the flux. Flux rates also may be referred to herein as flux velocities. Certain flux rates may represent, be equivalent to, be indicative of, or otherwise relate to rates of change of some process variables with respect to time. For example, the rate of the growth flux 132a may represent the rate of change of the process variable indicative of concentration or total amount of cellular biomass 130 disposed in the extracellular solution 124. The rate of a metabolite uptake flux 132b or 132c, at least under certain modeled bioreactor operating conditions, may represent the rate of change of the process variable indicative of amount or concentration of the corresponding metabolite in the extracellular solution 124. For example, the rate of oxygen uptake may represent the rate of change of the process variable indicative of extracellular oxygen concentration, while the rate of glucose uptake may represent the rate of change of the process variable indicative of glucose concentration. The velocities of fluxes corresponding to generation of target products or byproducts by the cellular biomass 130 may represent the rates of change of process variables associated with the amounts or concentrations of the corresponding target products or byproducts in the extracellular solution 124.

As noted above, in addition to the extracellular solution 124 and the cellular biomass 130, the computational model 104 may include descriptions of one or more virtual input streams 140 and/or one or more virtual output streams 142. Herein, the one or more input streams 140 may be referred to, for simplicity, as a singular "virtual input stream" or "input stream," and the one or more output streams 140 may be referred to, for simplicity, as a singular "virtual output stream" or "output stream." The descriptions of the streams 140, 142 may include variables indicating compositions and/or flow rates associated with the input stream 140 and output stream 142. In some implementations, the absolute value of a sum of volumetric flow rates of the liquid input stream 140 and the absolute value of a sum of volumetric flow rates of the output stream 142 are equal, leading to the constant volume of the extracellular solution. Implementations where the flow rates of the input stream 140 and output stream 142 flow rates are not balanced lead to a changing volume of the extracellular solution 124. The virtual input stream 140 and the virtual output stream 142 may affect the rates of change of process variables, including the ones indicative of extracellular solution 124 composition, for example, by replenishing the substrate metabolites and/or depleting the virtual contents of the modeled bioreactor 120.

Returning to the modeling process 100, the current values 150a of the process variables may serve as inputs into a kinetics model 160. The kinetics model 160 may compute a number of constraints 164 on the flux rates, i.e. the velocities of at least some of the fluxes 132a-i associated with the biomass 130. The constraints 164 may impose, for example, upper limits on flux rates that represent uptake of substrate metabolites. Consequently, the constraints 164 may constrain the rates of change of the process variables indicative of the amounts or concentrations of the substrate metabolites associated with the constrained fluxes. For example, the constraints may represent maximum uptake rates of oxygen, glucose, asparagine and/or glutamine and/or glycine, and, correspondingly, the maximum rates of change of process variables indicative of amounts or concentrations of oxygen, glucose, asparagine, glutamine and/or glycine in the extracellular solution 124 in the absence of the effects of the input 140 and output 142 streams.

The kinetics model 160 may compute the effects of current values 150a of process variables on metabolic reaction kinetics that, in turn, affect the constraints on flux rates. For example, as calculated by the kinetics model 160, the maximum uptake rates may depend on other process variables that are indicative or descriptive of physical or chemical properties of the extracellular solution 124. As a more specific example, process variables indicative of extracellular solution temperature, acidity or pH, and/or osmolarity may affect the maximum uptake rates.

The kinetics model 160 may compute the effects of the current values 150a of the process variables indicative of temperature, acidity, and/or osmolarity on the maximum metabolite uptake rates by weighing or multiplying optimal maximum uptake rates by process condition factors that account for deviations from optimal conditions. The optimal maximum uptake rates may correspond to the upper limits on the uptake rates under ideal process conditions. Process condition factors may then act as correction factors indicative of reduction in the uptake rates due to non-ideal conditions. The process condition factors may be defined in a variety of functional forms. For example, the process condition factors may have a value of unity for optimal process conditions for a given metabolite uptake, and decrease from unity with increasing deviation from the optimal process conditions. The process condition factors may take distinct or similar forms for different metabolite uptake rates, and the optimal conditions may be distinct or similar for different metabolite uptakes.

In some implementations, the kinetics model 160 computes the effects of the current values 150a indicative of concentrations of one or more metabolic byproducts on the metabolic reaction kinetics. For example, the concentrations of ammonia and lactate may reduce the maximum uptake rates of glucose, glutamine, asparagine and other substrate metabolites.

In some implementations, the kinetics model 160 models the effects of current values 150a of process variables on a rate constraint for intracellular metabolic flux responsible for cellular maintenance and the corresponding energy consumption rate. The cellular maintenance flux may model the metabolic pathway that consumes some of the energy available to the cellular biomass 130 in the form of ATP without growing the cellular biomass 132. The cellular maintenance flux, in some implementations, may be closely coupled with the flux that produces the target product. In other words, computing the constraint on the flux rate for the production of the target product may include computing the lower limit on the energy consumption rate for cellular maintenance.

To compute the lower limit on the energy consumption rate for cellular maintenance, the kinetics model 160 may account for the effect of the relevant current values 150a of the process variables. In some implementations, a "stress" variable may be defined to quantify how the process variables affect the lower limit on the energy consumption rate for cellular maintenance. Stress, in turn, may be a function of changes in extracellular solution temperature over time and the temperature differential that the cellular biomass 130 experiences or senses. In some implementations, stress is evaluated as a cumulative effect of the temperature differential integrated over time. Additionally or alternatively, in some implementations, different stress variables contribute to computing the minimum energy consumption rate for cellular maintenance, and/or stress variables account for the state of or changes in temperature, osmolarity, concentrations of byproducts, and/or high cellular density of the biomass 130.

Computing the effects of the current process variables 150a on flux rate constraints 164 by the kinetics model 160 may be based on experimental data. Mathematical formulas for the computation may be derived from theoretical models, may be empirical, and/or may combine empirical factors with theoretical derivations. In any case, the formulas for computing the effects of the current process variables 150a may include coefficients or model parameters that can be obtained with a calibration procedure, for example, by fitting calibration data. In some implementations the calibration data includes experimental data. Additionally or alternatively, computationally generated data may be used for at least some of the calibration. Fitting, regression, and/or optimization algorithms may aid in computing the kinetics model parameters from the calibration data.

In some implementations, before computing the effects of process variables on flux rate constraints 164, the kinetics model updates at least some of the process variables based on the control variables 152. The control variables may include temperature settings, acidity settings or any other suitable variables that may represent virtual control settings of the bioreactor 130. The modeling process 100 may receive the control variables 152 with associated times within the simulated time periods before starting the simulation, or may receive the control variables 152 at any time during the simulated time period.

The constraints 164 computed by the kinetics model 160, as discussed above, may define upper or lower limits on the rates of some of the fluxes 132a-i modeling the metabolic pathways of the virtual cellular biomass 130. The modeling process 100 may then use the constraints 164 to perform flux balance analysis 170 that simultaneously computes the flux rates 174 for all the modeled fluxes within the metabolic network of the cellular biomass 130. The flux balance analysis 170 may determine the flux rates that ensure that the concentrations of intracellular metabolites in the modeled biomass 130 are substantially at steady state. For example, if one of the fluxes consumes certain intracellular metabolites, while producing other metabolites, other fluxes may balance the first flux by consuming or removing all the metabolites produced by the first flux and producing or taking up from the extracellular solution 124 all the metabolites consumed by the first flux. The metabolites that are taken up by fluxes into the cellular biomass 130 may be removed from the extracellular solution 124, while the metabolites that are removed from the cellular biomass 130 by fluxes may be deposited into the extracellular solution 124. Consequently, while flux balance analysis may keep the rates of change of the intracellular concentrations or amounts of metabolites substantially at zero, it may contribute to the rates of change of the extracellular metabolites, which may include substrate metabolites, target products and byproducts, and the associated process variables.

Additionally or alternatively to contributing to computing changes in extracellular metabolite concentrations, flux balance analysis 170 may determine the rate of growth of the cellular biomass 130. In some implementations, the determined rate of growth may be negative, modeling cell death or attrition of the cellular biomass 130. The modeled death or attrition of the cellular biomass 130 may take place when the constraints on the flux rates include a lower limit on the energy consumption rate for cellular maintenance, and energy cannot be produced at the required rate by the metabolic fluxes in view of other flux rate constraints. Under this circumstance, the modeling process 100 may ignore the minimum maintenance energy constraint for flux balance analysis 170, and may compute a death rate based on the deficit in the maintenance energy production rate.

In addition to the constraints 164 computed by the kinetics model 160, flux balance analysis 170 may use a metabolic objective 172 (or, simply, metabolic objective) defined by the modeling process 100 to represent the biological objective of the virtual biomass 130. Without the metabolic objective function 172, the flux balance analysis 170 may not attain a unique solution, but may instead lead to a solution space with many or an infinite set of possible flux rates that keep intracellular metabolites in steady state. The metabolic objective function may therefore serve to restrict the flux balance analysis 170 to a unique solution. In other implementations, the solution to flux balance analysis 170 may not be unique even with the inclusion of the metabolic objective function 172.

A variety of metabolic objective functions 172 may be used in different implementations. For example, the metabolic objective function 172 may lead to a flux balance analysis 170 solution that maximizes the growth rate of the cellular biomass 130. Alternatively, the metabolic objective function 172 may lead to a flux balance analysis 170 solution that minimizes a linear combination of metabolite uptake rates. The linear combination may be a sum or a weighted sum of some or all of the uptake flux rates. In some implementations, a metabolic objective function combines a plurality of requirements, limitations, or constraints. For example, the metabolic objective function 172 may combine maximizing the growth rate while minimizing a linear combination of uptake flux rates. In another implementation, the metabolic objective 172 maximizes the flux associated with the cellular maintenance energy while minimizing the linear combination of the uptake flux rates.

Some or all of the flux rates 174 computed by flux balance analysis 170 may correspond to the rates of change of some of the process variables in the absence of contributions from the input stream 140 and output stream 142. The rates 174 may serve as input to a rate integration module 180 that may use the rates to compute updated values 150b of process variables. The updated values 150b of process variables then may become the current values 150a of process variables for the next time step.

The rate integration module 180 may compute the time rates of change of process variables using the flux rates 174 provided by the flux balance analysis module 170. In some implementations, the time rates of change of some of the process variables directly correspond to some of the flux rates 174. In other implementations, input and output flow variables 182 descriptive of the input stream 140 and the output stream 142 contribute to computing the rates of change of the process variables. The input and output flow variables 182 may contain information about the flow rates and compositions of the input stream 140 and the output stream 142. The contributions of the input stream 140 and the output stream 142 may be ignored when modeling a bioreactor 120 in batch operating mode. When modeling a bioreactor 120 in fed-batch operating mode, the flow variables 182 corresponding to the input stream 140 may contribute to the computation of the updated values 150b of process variables. When modeling a bioreactor 120 in continuous or perfusion operating modes, the flow variables 182 corresponding to both the input stream 140 and the output stream 142 may each contribute to the computation of the updated values 150b of process variables. In some implementations, when, for example, virtually harvesting the contents of the modeled bioreactor 120 without replenishing, only the flow variables 182 corresponding to the output stream 142 contribute to the rates of change of the process variables 150.

The flow variables 182 defining the input stream 140 and the output stream 142 that contribute to the computation of the updated set 150b of process variables may include the total volume flow rates of the streams 140, 142 (which may or may not be equal), as well as flow variables that define compositions of the input stream 140 and output stream 142. While the composition of the input stream 140 may be defined directly by input flow variables, i.e. independently of the computations performed in the modeling process 100, computing the composition of the output stream 142 may rely on the current values 150a of the process variables that describe the composition of the extracellular solution 124. Additionally or alternatively, flow variables 182 that contribute to the computation of composition of the output stream 142 may define the selectivity of the output filter 144. For example, glucose concentration of the input stream 140 may be defined directly as an input flow variable, while glucose concentration of the output stream 142 may be computed from the output flow variable indicative of the glucose selectivity of the output filter 144 and the process variable indicative of concentration of glucose in the extracellular solution 124. Analogously, for other metabolites or for the cellular biomass 130, the computations of concentrations in the output stream 142 may rely on the process variables indicative of corresponding concentrations in the extracellular solution 124, as well as the output flow variables indicative of the corresponding values of the output filter 144 selectivity.

When modeling bioreactor inhomogeneity, the input and output streams 140, 142 may represent the flows that connect different bioreactor portions (each modeled as a homogeneous bioreactor, e.g., bioreactor 120), as discussed herein. The input and output flow variables 182 may be computed, consequently, in view of a velocity field obtained from a physical model (e.g., a CFD model).

The rate integration module 180 may construct differential or difference equations using the flux rates 174, the input/output flow variables 182 that combine input flow variables and output flow variables, and the current values 150a of the process variables. The rate integration module 180 may subsequently compute the updated values 150b of the process variables using a numerical differential or difference equation solver. The time step 102 for integrating the rates may be equal to several seconds, several minutes, several hours, several days or any other suitable time period, depending on the implementation. Once it computes the updated values 150b of the process variables, the modeling process 100 may replace one or more of the current values 150a of the process variables with the updated values 150b. The replaced current values 150a may be discarded or saved in computer memory for further computations. The modeling process repeats for a next time step (e.g. the same duration as time step 102) with the new set of current values 150a, and repeats until the expiration of the simulated time period.

In an inhomogeneous bioreactor, current and updated values 150a,b of the process variables may be thought of as samples or representative values (for each bioreactor portion) of spatial distributions of the process variables. Thus, the differential or difference equations constructed by the integration module 180, may be partial differential or difference equations with the partial time derivatives, as described below. The integration module 180 may construct the spatial partial derivatives (or differences) based on process variable values in neighboring regions. The integration module 180 may combine the spatial partial derivatives of the process variable values with the velocity field (e.g., computed by a CFD model) to compute the updated values 150b of the process variables. In an example implementation, dot products between gradient vectors of process variables and local velocities may represent the difference between the input and output streams 140, 142 for a finite volume region representing a portion of the inhomogeneous bioreactor.

In some implementations, some of the process variables are updated based on the received values of control variables 152. In some implementations, the kinetics model 160 uses the control variables to update the values of the process variables before using the process variables to compute the constraints 164 on flux rates. Other parts of the modeling process also may update the process variables based on the control variables 152. The control variables may include temperature settings at different times of the simulated time period, times to change the filter 144, times to change rates and/or compositions of the input 140 and output 144 streams, or any other suitable variables that may represent virtual control settings of the bioreactor 130. In some implementations, the rate integration module 180 may construct and solve differential or difference equations representing a gradual response of the process variables to the corresponding control variables. For example, when a temperature control variable changes from one time step 102 to the next, the process variable for the temperature of the extracellular solution may be adjusted over multiple time steps by the rate integration module 180. In other implementations, the process variables instantly take on the values of the control variables, modeling a response that is quicker than the virtual time step 102.

The foregoing discussion elucidates and gives details of exemplary implementations of the kinetics model 160. As discussed above, the kinetics model 160 generates a plurality of constraints 164 on the flux rates by modeling one or more effects of at least some of the current values 150a of the process variables on metabolic reaction kinetics. The modeled effects may, for example, include the effect of temperature, acidity, and/or osmolarity on an upper limit of a metabolite uptake rate. The model may define an upper limit of a flux rate associated with a given metabolite uptake for some ideal conditions, which may include ideal temperature, ideal acidity, and/or ideal osmolarity. The kinetics model 160 may compute the effects of non-ideal conditions by multiplying the upper limit for ideal conditions by a correction factor. A correction factor indicative of a reduction in the uptake rate due to non-ideal conditions may be defined as a function of one or more variables, such as temperature, acidity, and/or osmolarity. In some implementations, the correction factor or includes an effect of concentration of a metabolic byproduct, such as lactate or ammonia.

Under ideal conditions, the upper limit of uptake rate for glucose, for example, may be modeled by the following equation:

$$v_{GLC} = \frac{\frac{vmax_{GLC}[GLC]}{Km_{GLC}}}{\left(1 + \frac{[GLC]}{Km_{GLC}}\right)}$$ (Equation 1),

where $v_{GLC}$ approaches a saturation value of $v_{maxGLC}$ when the conditions for the glucose uptake are ideal and when the glucose concentration in the extracellular solution is large enough that any further increase does not appreciably increase the glucose uptake rate. In Equation 1, $[GLC]$ is glucose concentration, and $Km_{GLC}$ is a kinetics model parameter describing the dependence of glucose uptake flux rate on glucose concentration. When the conditions are not ideal for glucose uptake, a correction factor, e.g., $C_{GLC}(T, pH, \pi, [LAC], [NH3]) < 1$ may multiply the glucose uptake rate, where the correction factor may depend on a variety factors including temperature, $T$, acidity, $pH$, osmolarity, $\pi$, lactate concentration, $[LAC]$, and/or ammonia concentration, $[NH3]$:

$$v_{GLC} = C_{GLC}(T, pH, \pi, [LAC], [NH3]) \frac{\frac{vmax_{GLC}[GLC]}{Km_{GLC}}}{\left(1 + \frac{[GLC]}{Km_{GLC}}\right)}$$ (Equation 2).

A correction factor may depend on 1, 2, 3, 4, 5 or any other suitable number of current values of process variables. A correction factor $C_{GLC}(T, pH)$ may represent correction that depends on temperature and acidity, while a correction factor $C_{GLC}(T, \pi, [NH3])$ may represent a correction that depends on temperature, osmolarity, and the concentration of ammonia. A correction factor depending on multiple variables may combine a plurality of correction factors depending on one or more variables. For example, $C_{GLC}(T, pH, \pi, [LAC])$ may combine four separately defined correction factors: a correction factor for temperature, $C_{GLC_T}(T)$, a correction factor for acidity, $C_{GLC_{pH}}(pH)$, a correction factor for osmolarity, $C_{GLC_\pi}(\pi)$, and a correction factor for lactate concentration $C_{GLC_{[LAC]}}([LAC])$, for example. The combination of constituent factors may take a variety of mathematical expressions that suitably represent the dependence of reaction kinetics on environmental factors in mechanistic models. For example, in some implementations, a correction factor dependent on multiple variables combines constituent correction factors by multiplication, as exemplified by the following equation:

$$C_{GLC}(T, pH, [LAC]) = C_{GLC_T}(T) \, C_{GLC_{pH}}(pH) \, C_{GLC_{[LAC]}}([LAC])$$ (Equation 3).

In another implementation, a correction factor dependent on multiple variables combines constituent correction factors in another manner:

$$C_{GLC}(T, pH) = 1 - k_{GLC_{T,pH}}\left(1 - C_{GLC_T}(T)\right)\left(1 - C_{GLC_{pH}}(pH)\right)$$ (Equation 4),

where $K_{GLC_{T,pH}}$ is a kinetics model 160 parameter for the correction. In the equation above, if $K_{GLC_{T,pH}} = 1$, the correction factor for temperature and the correction factor for acidity would both have to be zero to make the total correction factor zero as well.

The correction factors were described above as correction factors for glucose uptake merely for the purpose of illustration. A correction factor for glutamine uptake, asparagine uptake, or another metabolite uptake may have a similar form. In general, for any metabolite M, a correction factor may depend on multiple process variables including, for example, temperature, acidity, osmolarity, lactate concentration, ammonia concentration, and/or any other metabolite, generically designated $M2$, that may affect the maximum uptake flux rate for $M$. The resulting correction factor $C_M(T,pH,\pi,[LAC],[NH3],[M2])$ may combine separately defined correction factors: a correction factor for temperature, $C_{M_T}(T)$, a correction factor for acidity, $C_{M_{pH}}(pH)$, a correction factor for osmolarity, $C_{M_\pi}(\pi)$, a correction factor for lactate concentration $C_{M_{[LAC]}}([LAC])$, a correction factor for ammonia concentration $C_{M_{[NH3]}}([NH3])$, and a correction factor for the concentration of $M2$, $C_{M_{[M2]}}([M2])$.

Correction factors for single process variables may take a variety of mathematical forms that represent the mechanistic dependence of flux rate constraints on the environment. These correction factors may result from first principles derivations or empirical observations. A correction factor for temperature may take the following form:

$$C_M(T) = N(T - T_{min})\left(1 - e^{d_{temp}\,(T - T_{max})}\right) \qquad \text{(Equation 5)},$$

where the temperature is between minimum and maximum values such that $T_{min} < T < T_{max}$, $d_{temp}$ is a positive coefficient of dependence, and $N = 1/\max\{(T - T_{min})(1 - e^{d_{temp}\,(T - T_{max})})\}$ normalizes the correction factor to have a maximum value of unity. A correction factor for acidity may take the following form:

$$C_M(pH) = N\left|(1 - 10^{pH_{min} - pH})(1 - 10^{pH - pH_{max}})\right| \qquad \text{(Equation 6)},$$

where the acidity, represented by $pH$, is between minimum and maximum values such that $pH_{min} < pH < pH_{max}$, and $N = 1/\max\{|(1 - 10^{pH_{min} - pH})(1 - 10^{pH - pH_{max}})|\}$ normalizes the correction factor to have a maximum value of unity. A correction factor for osmolarity may take the following functional form:

$$C_M(\pi) = N\left(1 - \frac{1}{1 + e^{-(\pi - \pi_{0.5})/\pi_S}}\right) \qquad \text{(Equation 7)},$$

where $\pi > 0$ is osmolarity, $\pi_S$ and $\pi_{0.5}$ are coefficients that determine the dependence on osmolarity, and $N = 1 + e^{-\pi_{0.5}/\pi S}$ normalizes the correction factor to have a maximum value of unity. A correction factor for the effect of metabolite concentration may take the form of a saturation equation:

$$C_{M_{[M2]}}([M2]) = \left(1 + \frac{[M2]}{K_{m_{M2/M}}}\right)^{-1} \qquad \text{(Equation 8)},$$

where $[M2]$ is the concentration of a metabolite limiting the uptake flux and $K_{m_{M2/M}}$ is a coefficient that determines the dependence of the uptake flux rate limit of M on $[M2]$.

In an exemplary implementation, the maximum uptake rates for glucose, glutamine, asparagine, and oxygen, respectively, may be computed in the following manner:

$$v_{GLC} = C_{GLC}(T,pH)\frac{\frac{v_{max_{GLC}}[GLC]}{K_{m_{GLC}}}}{\left(1 + \frac{[LAC]}{K_{m_{GLC}}^{LAC}}\right)\left(1 + \frac{[GLC]}{K_{m_{GLC}}}\right)} \qquad \text{(Equation 9)},$$

$$v_{GLN} = C_{GLN}(T,pH)\frac{v_{max_{GLN_f}}\left(\frac{[GLN]}{K_{m_{GLN}}^{GLN}}\right) - v_{max_{GLN_r}}\left(\frac{[GLU]}{K_{m_{GLN}}^{GLU}}\right)\left(\frac{[NH_3]}{K_{m_{GLN}}^{NH3}}\right)}{1 + \left(\frac{[GLN]}{K_{m_{GLN}}^{GLN}}\right) + \left(\frac{[GLU]}{K_{m_{GLN}}^{GLU}}\right) + \left(\frac{[NH_3]}{K_{m_{GLN}}^{NH3}}\right) + \left(\frac{[GLU]}{K_{m_{GLN}}^{GLU}}\right)\left(\frac{[NH_3]}{K_{m_{GLN}}^{NH3}}\right)} \qquad \text{(Equation 10)},$$

$$v_{ASN} = C_{ASN}(T,pH) \frac{v_{max_{ASN_f}}\left(\frac{[ASN]}{Km_{\frac{ASN}{ASN}}}\right) - v_{max_{ASN_r}}\left(\frac{[ASP]}{Km_{\frac{ASN}{ASP}}}\right)\left(\frac{[NH_3]}{Km_{\frac{ASN}{NH3}}}\right)}{1 + \left(\frac{[ASN]}{Km_{\frac{ASN}{ASN}}}\right) + \left(\frac{[ASP]}{Km_{\frac{ASN}{ASP}}}\right) + \left(\frac{[NH_3]}{Km_{\frac{ASN}{NH3}}}\right) + \left(\frac{[ASP]}{Km_{\frac{ASN}{ASP}}}\right)\left(\frac{[NH_3]}{Km_{\frac{ASN}{NH3}}}\right)}$$ (Equation 11),

$$v_{O_2} = C_{O_2}(T,pH)\, v_{max_{O_2}}$$ (Equation 12),

where the correction factors due to non-ideal temperature and acidity are $C_{GLC}(T,pH)$, $C_{GLN}(T,pH)$, $C_{ASN}(T,pH)$, and $C_{O_2}(T,pH)$, the concentrations of glucose [GLC], glutamine [GLN], asparagine [ASN], lactate [LAC], glutamate [GLU], ammonia [NH$_3$], aspartate [ASP] affect some of the uptake rates, and the kinetics model 160 parameters for computing the limits of the uptake flux rates above are summarized in Table 1 below by relevant kinetic mechanism. In some implementations, the inputs to the equations are extracellular concentrations of metabolites, and in some implementations the inputs to the equations are intracellular concentrations. In other implementations, a combination of extracellular and intracellular concentrations of metabolites can be inputs to computing the constraints 164 on flux rates. In some implementations, additional kinetics model 160 parameters quantify the dependence of uptake flux rates on temperature, acidity, and combined correction factors.

**Table 1. Kinetics model parameters**

| Kinetic mechanism | Model Parameters |
|---|---|
| temperature dependence | $d_{temp}; T_{max}; T_{min}$ |
| pH dependence | $pH_{min}; pH_{max};$ |
| osmolarity dependence | $\pi_{k0.5}; \pi_{slope}$ |
| glucose kinetics | $K_{m_{\frac{GLC}{LAC}}}$<br>$v_{max_{GLC}}; K_{m_{GLC}};$ |
| glutamine kinetics | $K_{m_{\frac{GLN}{GLN}}};$<br>$K_{m_{\frac{GLN}{GLU}}};$<br>$K_{m_{\frac{GLN}{NH3}}}$<br>$v_{max_{GLN_f}}; v_{max_{GLN_r}};$ |
| asparagine kinetics | $K_{m_{\frac{ASN}{ASN}}};$<br>$K_{m_{\frac{ASN}{ASP}}};$<br>$K_{m_{\frac{ASN}{NH3}}}$<br>$v_{max_{ASN_f}}; v_{max_{GLN_r}};$ |
| oxygen kinetics | $q_{O_2 max}$ |

Additionally or alternatively to calculating the constraints on metabolite uptake flux rates, the kinetics model 160 may calculate constraints on one or more flux rates for metabolic processes that determine energy consumption for cellular maintenance and/or generation of a target product. While the constraints on the metabolite uptake rates may be upper limits on the uptake rates, the constraint on maintenance energy may be a lower limit of the associated flux rate. The maintenance energy represents the modeled metabolic requirement of the virtual cellular biomass 130 that may reflect the energy used by real cells to survive in a real environment. The energy may be represented by the intracellular concentration or amount of ATP. When cellular biomass 130 produces more energy or ATP than needed for maintenance, the cellular biomass may grow with a positive growth rate. On the other hand, when the cellular biomass 130 fails to produce the amount of ATP or energy needed for maintenance, the cellular biomass 130 may begin decreasing or virtually dying. The modeling process 100 may account for the decrease in cellular biomass 130 due to cell death or attrition by assigning a negative growth rate to an equation for growth.

The lower bound of cellular maintenance energy required to prevent the decrease in cellular biomass may depend on a variety of environmental factors represented by one or more process variables. The kinetics model 160 may compute a stress variable or factor, $S$, to represent at least some of the environmental factors affecting the maintenance energy requirement. The modeling process 100 may then compute the minimum maintenance energy, as the minimum rate of ATP consumption as

$$v_{ATP} = v_{ATP,min}\left(1 + \frac{S}{k_{S,mnt}}\right) \qquad \text{(Equation 13),}$$

**where** $v_{ATP,min}$ represents the maintenance energy requirement when there is no stress, or $S = 0$, and $k_{S,mnt}$ is a coefficient adjusting the influence of the stress variable. While the equation above represents a linear dependence of the maintenance energy requirement on $S$, the kinetics model 160 may use other functional forms including polynomial, square root or other fractional power, logarithmic, exponential, or a combination of the preceding forms, or any other suitable relationship. The stress variable itself may be calculated based on temperature, acidity and/or any other suitable process variables and their changes over time. The stress variable may include a dependence on concentrations of metabolites, such as metabolic byproducts. In some implementations, however, the kinetics model 160 may separately include effects of concentrations of some metabolites in computing the minimum maintenance energy, as expressed by the exemplary equation below:

$$v_{ATP} = v_{ATP,min}\left(1 + \frac{S}{k_{S,mnt}}\right) + k_{mnt}\left(1 + \frac{K_{mnt,NH_3}}{[NH_3]}\right)^{-1}\left(1 + \frac{K_{mnt,LAC}}{[LAC]}\right)^{-1} \qquad \text{(Equation 14),}$$

where $k_{mnt}$ is the maximum additional maintenance energy or ATP consumption rate approached when significant lactate concentration $[LAC] \gg K_{mnt,LAC}$ and ammonia concentration $[NH_3] \gg K_{mnt,NH_3}$ are present in the extracellular solution (or in intracellular solution, in some implementations), and $K_{mnt,LAC}$, $K_{mnt,NH_3}$ are kinetics model parameters determining the influence of lactate and ammonia, respectively, on minimum maintenance energy. While the equation above shows a single additive term accounting for the influence of two metabolites, separate additive terms or any another suitable functional dependence may account for metabolite concentrations or other environmental factors affecting minimum maintenance energy or minimum ATP consumption rate for cellular maintenance.

After computing the lower bound on maintenance energy, the kinetics model 160 may additionally compute the minimum rate of generating a target product. The target product may be an antibody or another suitable biologic product. The kinetics model 160 may compute the lower constraint for generation of target product by using a proportional relationship between the minimum maintenance energy and product generation and applying correction factors that account for non-ideal conditions for product generation. For example, the kinetics model 160 may compute the minimum rate of product generation, where the target product is an antibody, by the following equation:

$$v_{ANTI} = C_{ANTI}(T, pH) \cdot \beta \left(1 + \frac{[LAC]}{K_{i,ANTI/LAC}}\right)^{-1} v_{atp} \qquad \text{(Equation 15),}$$

where $C_{ANTI}(T,pH)$ is a correction factor that accounts for the reduction of antibody generation due to non-ideal temperature and acidity, $[LAC]$ is lactate concentration, $K_{i,ANTI/LAC}$ is a kinetics model parameter reflecting the inhibitory effect of lactate on product formation, and $\beta$ is a proportionality constant relating the rate of ATP consumption for maintenance and the rate of antibody production. The correction factor $C_{ANTI}(T,pH)$ may have a similar functional dependence on temperature and acidity as the correction factors for the metabolic uptake rates. Other corrections factors that, for example, account for additional inhibitory effects of metabolites may be included in the computation of the lower limit of antibody or another target product generation.

Kinetics model parameters, such as the ones used in Equations 1-15 and listed in Table 1, may be obtained using a calibration procedure or, simply, calibration. Experiments conducted for the purpose of calibration can supply experimental calibration data. The experiments for calibration may be perfusion experiments, small scale batch or chemostat experiments, and/or any other suitable experiments. Additionally or alternatively, calibration data may be adapted from published literature, from theoretical computations, and/or any suitable combination of sources. The calibration procedure may apply a variety of regression, fitting, or optimization techniques and/or algorithms, including, for example, Levenberg-Marquardt, differential evolution, and/or genetic algorithms, to the calibration data to find the kinetics model parameters. The calibration procedure may combine global and local optimization algorithms, and may use one or more of a variety of suitable cost or objective functions. The calibration procedure may compute the kinetics model parameters separately and prior to the execution of the modeling process 100. In some implementations, however, one or more parts of the calibration procedure are integrated into the kinetics model 160. Furthermore, in some applications, different sets of kinetics model parameters are applied at different stages of the simulation, as determined, for example, by the current values of the process variables 150a.

Experimental calibration data may include concentrations at different points in time of a set of metabolites. An aggregate calibration error to be minimized, may be defined as

$$\varepsilon = \sum_k w_k \sum_t \left( \frac{[M_k]_{sim}(t) - [M_k]_{exp}(t)}{[M_k]_{exp}(t)} \right)^2 \qquad \text{(Equation 16)},$$

where $[M_k]_{sim}(t)$ is the simulated concentration at time $t$ of a $k$-th metabolite, $[M_k]_{exp}(t)$ is the experimental concentration at time t of a k-th metabolite, and $w_k$ is the weight of the error for the $k$-th metabolite in the aggregate error. In this implementation, the $k$-th metabolite error is computed as the sum of squares of relative concentration errors at different times. In other implementations, the error may be based on a combination of absolute and relative concentration errors. The aggregate error is a weighted sum of errors for each metabolite. The weights for the different metabolites may emphasize the importance of some metabolite concentrations in the calibration while suppressing or excluding other metabolite concentrations, and may depend on the experiment. For example, concentration of glucose may be more pertinent to an experiment that corresponds to the growth stage of a bioprocess, while an antibody concentrations may be more pertinent to an experiment that corresponds to the production stage. In general, a variety of suitable experiments and error function formulations may be appropriate for calibrating different kinetics model parameters.

The following discussion elucidates and gives details of exemplary implementations of the flux balance analysis 170. Flux balance analysis 170 uses the constraints 164 generated by the kinetics model 160, such as, for example, the maximum metabolite uptake rates, the minimum energy consumption rate for cellular maintenance and the associated minimum rate of target product generation, to compute a complete set of flux rates 174 for the metabolic model of the cellular biomass 130. Different metabolic models may account for different metabolites and a different arrangement of metabolic pathways. Each metabolic pathway flux may represent a reaction or a set of reactions collectively converting a set of metabolites into another set of metabolites. The proportions in which a metabolic pathway flux consumes or produces metabolites form stoichiometric coefficients for the flux. Table 2 below illustrates an exemplary network of metabolic fluxes modeling CHO cells configured for producing an antibody product.

**Table 2. Exemplary Network of Metabolic Pathway Fluxes**

| # | Metabolic pathway flux |
|---|---|
| 1 | $ATP_c + H_2O \rightarrow ADP_c + H_c + P_{Ic}$ |
| 2 | $0.5\ FAD_c + 0.5\ H_c + NADH_c \rightarrow 0.5\ FADH_{2c} + 0.5\ NAD_c + 0.5\ NADH_m$ |
| 3 | $GL\ U_c \rightarrow GL\ U_e$ |
| 4 | $2.0\ CYS_e + 3.0\ H_e + O_{2c} \rightarrow CC_e + 2.0 H_2O$ |
| 5 | $\rightarrow O_{2c}$ |
| 6 | $NH_{3c} \rightarrow NH_{3e}$ |
| 7 | $SER_e \rightarrow SER_c$ |
| 8 | $GLY_e \leftrightarrow GLY_c$ |
| 9 | $GLN_e \leftrightarrow GLN_c$ |
| 10 | $CC_e + GLU_c \rightarrow CC_c + GLU_e$ |
| 11 | $ASP_e \leftrightarrow ASP_c$ |
| 12 | $ASN_e \rightarrow ASN_c$ |
| 13 | $ALA_e \leftrightarrow ALA_c$ |
| 14 | $H_e + LAC_e \leftrightarrow H_c + LAC_c$ |
| 15 | $ATP_c + GLC_e \rightarrow ADP_c + G6P_c + H_c$ |
| 16 | $ANTI_c \rightarrow ANTI_e$ |
| 17 | $BIOM_c \rightarrow BIOM_e$ |

(continued)

| # | Metabolic pathway flux |
|---|---|
| 18 | $0.045\ ALA_c + 0.038\ ASN_c + 0.038\ ASP_c + 4.032\ ATP_c + 0.027\ CYS_c + 0.045\ GLN_c + 0.048\ GLU_c + 0.128\ GLY_c + 10.43\ H_2O + 0.101\ SER_c \rightarrow ANTI_c + 4.032\ ADP_c + 5.3\ H_c + 4.032\ P_{Ic}$ |
| 19 | $0.084\ ALA_c + 0.041\ ASN_c + 0.08\ ASP_c + 3.78\ ATP_c + 0.026\ CYS_c + 0.004\ FAD_c + 0.452\ G6P_c + 0.087\ GLN_c + 0.056\ GLY_c + 3.78\ H_2O + 0.639\ NAD_c + 0.427\ OAA_c + 0.096\ SER_c \rightarrow BIOM_c + 3.78\ ADP_c + 0.004\ FADH_{2c} + 0.008\ GLU_c + 3.705\ H_c + 0.445MAL_c + 0.639\ NADH_c + 0.452\ P_{Ic} + 0.209\ PYR_c$ |
| 20 | $1.5\ ADP_c + FADH_{2c} + 1.5\ H_c + 0.5\ O_{2c} + 1.5\ P_{Ic} \rightarrow 1.5\ ATP_c + FAD_c + 2.5\ H_2O$ |
| 21 | $2.5\ ADP_c + 3.5\ H_c + NADH_m + 0.5\ O_{2c} + 2.5\ P_{Ic} \rightarrow 2.5\ ATP_c + 3.5\ H_2O + NAD_m$ |
| 22 | $CC_c + NADH_c \rightarrow 2.0\ CYS_c + NAD_c$ |
| 23 | $CO_{2c} + H_c + NADH_c + NH_{3c} + SER_c \leftrightarrow 2.0\ GLY_c + H_2O + NAD_c$ |
| 24 | $AKG_c + ASP_c \rightarrow GLU_c + OAA_c$ |
| 25 | $ASN_c + H_2O \leftrightarrow ASP_c + H_c + NH_{3c}$ |
| 26 | $AKG_c + 2.0\ H_c + NADH_m + NH_{3c} \leftrightarrow GLU_c + H_2O + NAD_m$ |
| 27 | $GLN_c + H_2O \leftrightarrow GLU_c + H_c + NH_{3c}$ |
| 28 | $MAL_c \rightarrow CO_{2c} + H_c + PYR_c$ |
| 29 | $MAL_c + NAD_m \rightarrow H_c + NADH_m + OAA_c$ |
| 30 | $ADP_c + AKG_c + FAD_c + H_2O + NAD_m + P_{Ic} \rightarrow ATP_c + CO_{2c} + FADH_{2c} + MAL_c + NADH_m$ |
| 31 | $H_2O + 2.0\ NAD_m + OAA_c + PYR_c \rightarrow AKG_c + 2.0\ CO_{2c} + H_c + 2.0\ NADH_m$ |
| 32 | $GLU_c + PYR_c \leftrightarrow AKG_c + ALA_c$ |
| 33 | $H_c + NADH_c + PYR_c \leftrightarrow LAC_c + NAD_c$ |
| 34 | $3.0\ ADP_c + G6P_c + 2.0\ NAD_c + 2.0P_{Ic} \rightarrow 3.0\ ATP_c + 2.0\ H_2O + H_c + 2.0\ NADH_c + 2.0\ PYR_c$ |

The subscripts distinguish cytosol or cellular metabolites (c), extracellular metabolites (e), and mitochondrial metabolites (m) that are part of cellular processes. Table 3 below lists and describes the metabolites that take part in the metabolic fluxes in Table 2.

### Table 3. Metabolite Definitions

| Metabolite ID | Metabolite Name | Metabolite Formula |
|---|---|---|
| ADP | ADP | $C_{10}H_{12}N_5O_{10}P_2$ |
| AKG | 2-Oxoglutarate | $C_5H_4O_5$ |
| ALA | L-Alanine (or just Alanine) | $C_3H_7NO_2$ |
| ANTI | Antibody (Target product in this network) | $C_{4.81}N_{1.28}O_{2.51}H9.44$ |
| ASN | L-Asparagine (or just Asparagine) | $C_4H_8N_2O_3$ |
| ASP | L-Aspartate (or just Aspartate) | $C_4H_6NO_4$ |
| ATP | ATP | $C_{10}H_{12}N_5O_{13}P_3$ |
| BIOM | Biomass | $C_{5.68}N_{1.40}O_{2.92}H_{11.49}P_{0.09}$ |
| CC | Cystine | $C_6H_{13}N_2O_4S_2$ |
| $CO_2$ | Carbon Dioxide | $CO_2$ |
| CYS | L-Cysteine (or just Cysteine) | $C_3H_7NO_2S$ |
| FAD | Flavin adenine dinucleotide oxidized | $C_{27}H_{31}N_9O_{15}P_2$ |
| $FADH_2$ | Flavin adenine dinucleotide reduced | $C_{27}H_{33}N_9O_{15}P_2$ |
| G6P | D-Glucose 6-phosphate | $C_6H_{11}O_9P$ |

(continued)

| Metabolite ID | Metabolite Name | Metabolite Formula |
|---|---|---|
| GLC | D-Glucose | $C_6H_{12}O_6$ |
| GLN | L-Glutamine (or just Glutamine) | $C_5H_{10}N_2O_3$ |
| GLU | L-Glutamate (or just Glutamate) | $C_5H_8NO_4$ |
| GLY | Glycine | $C_2H_5NO_2$ |
| H | H+ | H |
| $H_2O$ | Water | $H_2O$ |
| LAC | L-Lactate (or just Lactate) | $C_3H_5O_3$ |
| MAL | L-Malate (or just Malate) | $C_4H_4O_5$ |
| NAD | Nicotinamide adenine dinucleotide | $C_{21}H_{26}N_7O_{14}P_2$ |
| NADH | Nicotinamide adenine dinucleotide - reduced | $C_{21}H_{27}N_7O_{14}P_2$ |
| $NH_3$ | Ammonia | $H_3N$ |
| $O_2$ | Oxygen | $O_2$ |
| OAA | Oxaloacetate | $C_4H_2O_5$ |
| $P_I$ | Phosphate | $HO_4P$ |
| PYR | Pyruvate | $C_3H_3O_3$ |
| SER | L-Serine (or just Serine) | $C_3H_7NO_3$ |

Flux balance analysis 170 of the modeling process 100 may compute the flux rates for each of the metabolic pathways. Continuing with the example of the metabolic network described in Table 2, flux balance analysis 170 may determine a flux rate for each of the 34 metabolic pathways, subject to a number of conditions, limitations, constraints, or objectives. One constraint for the computation of the fluxes may be the requirement to maintain the steady-state of the cellular biomass 130 metabolite concentrations. For example, metabolic pathway 33 in Table 2 may convert, in equal proportions indicated by the unity stoichiometric coefficients, hydrogen, reduced nicotinamide adenine dinucleotide, and pyruvate into the non-reduced form of nicotinamide adenine dinucleotide and lactate. The set of other metabolic fluxes may then produce hydrogen, reduced nicotinamide adenine dinucleotide and consume the non-reduced form of nicotinamide adenine dinucleotide and lactate. As another example, metabolic pathway 14 of Table 2 may either produce hydrogen by taking it up from the extracellular solution 124 or consume lactate by removing it into the extracellular solution 124, but it may not accomplish both. There may not be a pathway for taking up pyruvate from the extracellular solution 124 or removing pyruvate into the extracellular solution 124, leading to the requirement that pyruvate may need to be balanced with intracellular pathway fluxes. In general, intracellular metabolite concentrations may be balanced in steady state with intracellular pathway fluxes or interactions with the extracellular solution. The equation $\bar{\bar{S}} \cdot \bar{v} = 0$, where $\bar{\bar{S}}$ is the stoichiometric matrix of the metabolic network and a velocity vector, $\bar{v}$, is the vector of all the flux rates, describes the steady state condition for flux balance analysis 170. The steady state condition, however, may not be sufficient to find a unique solution to flux balance analysis 170 and, therefore, flux balance analysis 170 may require other constraints 164 to limit the set of possible solutions.

Some of the metabolic pathways account for removal of metabolites from the cellular biomass 130 or uptake of metabolites into the cellular biomass 130 from the extracellular solution 124. The flux balance analysis 170 steady state solution may depend on the constraints for uptake or removal flux rates. For example, uptake fluxes of substrate metabolites may be constrained by the concentration of the corresponding metabolites in the extracellular solution 124 and by non-ideal environmental conditions, as discussed above with reference to the kinetics model 160. For the metabolic network described by Table 2, for example, oxygen may be in abundance in the extracellular solution, as indicated by metabolic pathway 5 of Table 2 which omits extracellular oxygen input. Nevertheless, the maximum uptake rate of oxygen may be constrained by Equation 12, which takes into account the maximum uptake rate for ideal conditions and the non-ideal condition correction factor indicated by the current values 150a of the process variables. In some implementations, at least some of the metabolic flux rates corresponding to metabolic pathways 3-17 of Table 2 are constrained by the rate constraints 164 computed by the kinetics model 160. In other implementations, the rate constraints 164 computed by the kinetics model 160 apply to intracellular metabolic pathways that may be indirectly affected by extracellular conditions and uptake rates. For example, the glutamine constraint of Equation 10 may limit the flux rate of metabolic pathway 27, the glucose constraint of Equation 9 may limit the flux rate of metabolic pathway 34, and the asparagine constraint of Equation 11 may limit the flux rate of metabolic pathway 25.

Flux balance analysis 170 may be unable to compute a unique set of flux rates for the steady state condition under constraints 164 generated by the kinetics model 160 and may additionally obtain at least one metabolic objective 172 defined for the cellular biomass 130. The metabolic objective for the virtual cellular biomass 130 in the *in silico* model 104 may correspond to a real biological objective of an *in vivo* cellular culture. A variety of metabolic objective functions may constrain the flux balance analysis 170 in different implementations, representing different biological alternatives. The metabolic objective function 172 may maximize the growth rate of the cellular biomass 130, minimize a linear combination of metabolite uptake rates, maximize flux rates for pathways associated with energy or ATP production, or generate another limitation on the set of flux rates. In general, the metabolic objective function 172 can compute a cost or value for any set of flux rates represented by the velocity vector, as may be represented by a velocity vector $\bar{v}$ (set of flux rates) of a metabolic network. In some implementations, the flux balance analysis 170 minimizes the cost or value, while in other implementations the flux balance analysis 170 maximizes the cost or value. Moreover, the metabolic objective function may depend on a variety of current values 150a of process variables, their history, and a variety of other variables describing the cellular biomass 130 and its environment at a current time and at previously simulated times.

Considering a collection of $N$ metabolic pathways or reactions with flux rates $v_1$, $v_2$, ... $v_{N-1}$, $v_N$ that are the elements of the velocity vector $\bar{v}$, the metabolic objective function 172 can take a variety of mathematical expressions, exemplified in Table 4 below. The table also provides explanations and motivation for the metabolic objective functions.

**Table 4. Metabolic Objectives**

| Objective # | Objective Expression | Explanation |
|---|---|---|
| 1 | $\min \sum_{i=1}^{N} |v_i|$ | Minimizing sum of flux magnitudes may approximate minimizing enzyme activity. |
| 2 | $\max v_{BIOM}$ | Maximizing biomass growth |
| 3 | $\max \dfrac{v_{BIOM}}{\sum_{i=1}^{N} |v_i|}$ | Combining 1 and 2, maximizing growth per flux. |
| 4 | $\max v_{atp}$ | Maximizing the maintenance flux rate |
| 5 | $\min \dfrac{a_0 + \sum_{i=1}^{N} a_i v_i}{b_0 + \sum_{i=1}^{N} b_i v_i}$ | Flexible expression for minimizing or maximizing a ratio of linear combinations of flux rates |
| 6 | $\min \dfrac{a_0 + \sum_{i=1}^{N} a_i v_i^2}{b_0 + \sum_{i=1}^{N} b_i v_i^2}$ | Flexible expression for minimizing or maximizing a ratio of linear combinations of squared flux rates |

The flux balance analysis 170 may be unable to find a solution that satisfies all the constraints generated by the kinetics model 160 for a given metabolic objective function 172. Specifically, if the constraint on the minimum rate of the maintenance flux cannot be satisfied for the objective function 172 that maximizes growth or a ratio of growth to some function of other flux rates, the flux balance analysis 170 may switch to a different metabolic objective 172 and may ignore the constraints on minimum maintenance flux rate and minimum production rate of a target product. The flux balance analysis 170 may switch to a metabolic objective 172 that maximizes the maintenance flux rate, for example. Upon finding a steady-state solution that maximizes the maintenance flux rate, while satisfying the remaining constraints 164, such as the maximum uptake rates, the flux balance analysis 170 may compute the death rate or attrition rate for the cellular biomass 130 based on the difference between the ignored minimum maintenance flux rate and the achieved maintenance flux rate. The death rate equation representing the rate of change of the biomass 130, relative to the biomass concentration, may be computed using

$$v_d = c_d \left( v_{ATP,min} - v_{ATP} \right) \qquad \text{(Equation 17),}$$

where $v_{ATP,min}$ - $v_{ATP}$ a positive difference between the minimum maintenance flux rate and the computed insufficient maintenance flux rate, and $c_d$ is a proportionality coefficient that is a parameter of the modeling process 100.

The following discussion elucidates and gives details of exemplary implementations of the rate integration module 180. Once the flux balance analysis 170 computes the flux rates 174, including the growth or death rate of the cellular biomass 130, the rate integration module 180 may use the computed flux rates 174 to compute the updated values 150b of the process variables. The rate integration module 180 may perform mass balance for the virtual contents of the bioreactor 120. Additionally or alternatively, the rate integration module 180 may compute the consumption of bioreactor 120 inputs, including substrate metabolites, and/or the production of the target product.

The rate integration module 180 may first compute the time rates of change of the process variables and then solve or numerically integrate the differential or, in the discrete time form, the difference equations for the process variables. The equations for the time rates of change of the process variables may include contributions from the input stream 140 and output stream 142. Some exemplary equations for a set of process variables are shown below. For example, a general equation for the time rate of change of a metabolite in the extracellular solution may be written as

$$\frac{d[M_e]}{dt} = [BIOM] \sum_{i=1}^{N} S_{M_e,i} v_i + \frac{1}{V}\left(F_{IN} C_{IN_M} - F_{OUT} T_M [M_e]\right) \qquad \text{(Equation 18)},$$

where $[M_e]$ is the concentration of the metabolite in the extracellular solution 124 (i.e., the process variable under consideration), $[BIOM]$ is the concentration of biomass in the extracellular solution 124, $S_{M_e,i}$ is the stoichiometric coefficient for the extracellular metabolite $M_e$ in a metabolic pathway flux $i$, $v_i$ is the flux rate for flux $i$ as computed by the flux balance analysis 170, $V$ is the volume of the extracellular solution 124, $F_{IN}$ is the volume flow rate of the input stream 140, $C_{IN_M}$ is the concentration of metabolite M (the same substance as the extracellular metabolite $M_e$) in the input stream 140, $F_{OUT}$ is the volume flow rate of the output stream 142, and $T_m$ is the transmission or selectivity factor of filter 144 for metabolite M. The expression $\sum_{i=1}^{N} S_{M_e,i} v_i$ is the sum contribution to the extracellular concentration of $M_e$ by all of the fluxes in the metabolic network and may simplify to $-v_M$ or $v_M$ when, correspondingly, only an uptake flux or only a removal (excretion) flux affects the extracellular metabolite concentration of $M_e$. In implementations of the modeling process 100 with multiple input streams, a summation over multiple terms that represent the multiple input streams may replace $F_{IN} C_{IN_M}$. The volume V, may be a process variable with the rate of change given by

$$\frac{dV}{dt} = F_{IN} - F_{OUT} \qquad \text{(Equation 19)},$$

and in implementations that keep the volume substantially constant, $F_{IN} = F_{OUT}$. In some implementations, there is no filter 144 for the output stream 142 and $T_M = 1$ for every metabolite, while in other implementations the filter does not substantially filter a given metabolite, leading to $T_M = 1$ for a given metabolite.

For inhomogeneous reactors, Equation 18 may represent a rate of change of a metabolite in a given portion of a bioreactor and be adjusted to account for inhomogeneity and for mixing dynamics, as discussed herein. In some implementations, particularly with comparatively fine spatial discretization, the additive term representing input and output streams 140, 142 may be replaced by a dot product of a concentration gradient for the metabolite with the velocity field. Such an approach, in a sense, computes the total derivative with respect to time based on the partial derivative with respect to time and the partial derivatives with respect to spatial dimensions. In other implementations, the additive term representing input and output streams 140, 142 may be a result of adding flow contributions from neighboring portions of the inhomogeneous bioreactor in view of a velocity field. In some bioreactor volume discretizations, one portion may include the input stream that comes in from outside of the reactor, while another portion may include the output stream from the reactor.

Returning to the discussion of a homogeneous bioreactor model, though, as discussed, without losing general applicability to inhomogeneous bioreactors, Equation 18 may be adapted to apply to one or more specific metabolites. Considering, for example, an implementation of a modeling process 100 where the cellular biomass 130 is modeled by the metabolic pathways in Table 2, with an input stream 140 and an output stream 142 having flow rates $F_{IN} = F_{OUT} = F$, a glucose concentration in the input stream 140 of $C_{IN_{GLC}}$ and a perfusion configuration with $T_{GLC} = T_{ANTI} = 1$ and $T_{BIOM} = 0$, the equations for the time rate of change for the concentrations of glucose, biomass, and the antibody product may be:

$$\frac{d[GLC]}{dt} = -[BIOM]v_{15} + \frac{F}{V}\left(C_{IN_{GLC}} - [GLC]\right) \qquad \text{(Equation 20)},$$

$$\frac{d[ANTI]}{dt} = [BIOM]v_{16} - \frac{F}{V}[ANTI] \qquad \text{(Equation 21)},$$

$$\frac{d[BIOM]}{dt} = [BIOM]v_{17} \qquad \text{(Equation 22)},$$

where $v_{15}$ is the flux rate for pathway 15 for glucose uptake, $v_{16}$ is the flux rate for pathway 16 for antibody removal, and $v_{17}$ is the flux rate for pathway 17 for biomass removal. While there may not be a distinction in some implementations between intracellular biomass and extracellular biomass, the definitions in Table 2 separate the biomass generation pathway 19 from the biomass removal pathway 17, with the extracellular biomass concentration representing the cellular biomass 130 in the extracellular solution 124.

The rate integration module 180 may compute the time rate of change of the cellular biomass 130 based on death or attrition rate computed in the flux balance analysis 170 as

$$\frac{d[BIOM]}{dt} = -[BIOM]v_d \qquad \text{(Equation 23)},$$

where [BIOM] is the concentration of the cellular biomass 130 in the extracellular solution 124, and $v_d$ is the death rate or attrition rate computed in the flux balance analysis 170. While biomass 130 may only account for virtual living cellular biomass with ongoing metabolic processes, in some implementations, the concentration or amount of dead cells or dead biomass is another one of the process variables that is updated by the modeling process 100. Any decrease in the virtual living biomass 130 represented by [BIOM] may contribute to a corresponding increase in a value of the process variable representing dead biomass. While dead biomass may not have ongoing metabolic processes, it may affect the properties of the extracellular solution 124.

The rate integration module 180 may compute total amounts of substrate metabolites supplied through one or more input streams 140 and consumed by the cellular biomass 130 and a total amount of product removed or collected through one or more output streams 142, throughout the duration of the time period simulated by the modeling process 100. For example, the collected antibody product may be computed using

$$\frac{dANTI}{dt} = T_{ANTI} F_{OUT} [ANTI] \qquad \text{(Equation 24)},$$

Where ANTI is the amount in moles or mass of the antibody product collected, $T_{ANTI}$ is a time-varying transmission of the antibody by the output filter 144, $F_{OUT}$ is a time varying flow rate of the output stream 142, and [ANTI] is molar concentration or density of the antibody in the extracellular solution 124. The flow rate and transmission may vary over time to represent different regimes of operation or to simulate output filter performance and may be represented by process variables that are updated by the rate integration module 180. The regimes of operation may represent an accumulation stage of the product in the extracellular solution 124 and the harvesting stage that initiates once the product concentration is at a sufficient level. The separate accumulation and harvesting stages in real bioreactor operation may reduce the cost of separating the product from the output stream 142. The filter transmission function $T_{ANTI}$ may represent step changes in transmission when the filter 144 is replaced or gradual changes in transmission due to retention of some of the filtered metabolites or other components of the output stream 142.

In some implementations, the rate integration module 180 may compute the stress on the cellular biomass 130 from changes in temperature, pH, and/or other process variables. These changes in the process variables may depend on the control variables 152. The control variables 152, in some implementations, are processed by the flux balance analysis 170 and/or rate integration module 180. Stress may accumulate over time, making the rate integration module 180 suitable for computing the updated process variable indicative of stress. Additional process variables may facilitate the computation of stress. For example, a temperature memory variable may indicate a time evolution of an effect of shifting temperature on the cellular biomass 130 maintenance energy requirement.

The rate integration module 180 may use a computed time rate of change for a metabolite concentration to compute an updated value of the corresponding process variable for the concentration, based on the current value of the process variable, in the following manner:

$$[M_e]_{n+1} = [M_e]_n + \frac{d[M_e]}{dt} \Delta t_{n+1} \qquad \text{(Equation 25)},$$

where $[M_e]_{n+1}$ may represent the updated value of extracellular concentration of metabolite $M$ after the $(n + 1)^{th}$ time step 102 of the modeling process 100, $[M_e]_n$ may represent the current value after the $n^{th}$ time step 102, $\frac{d[M_e]}{dt}$ is the computed derivative, and $\Delta t_{n+1}$ is the duration of the $(n + 1)^{th}$ time step.

For inhomogeneous reactors, $\frac{d[M_e]}{dt}$ may represent the total derivative for a portion of the bioreactor, computed, for example, in view of gradients in concentration and a velocity field, as discussed above. The velocity field may be updated at every time step 102, more frequently than the time step 102, or less frequently than the time step 102. The velocity field may be updated in response to a certain trigger condition in the simulation. For example, a change in flow rates of simulated streams supplying the bioreactor, a change in simulated mixing of the bioreactor, or a substantial change in a physical property of bioreactor contents may trigger a new computation of the velocity field.

In some implementations, the duration of each time step 102 is kept constant, while in other implementations the modeling process can change the duration of the time step 102 for different values of *n*. The change in the duration of each time step 102 may depend, for example, on the computed flux rates 174, on the flow rates of the input stream 140 or output stream 142, on the control variables 152, and/or on the requirements regarding the speed or precision of execution of the modeling process 100.

The rate integration module 180 may use one or more of a variety of techniques for integrating the derivatives or the rates of change of the process variables to implement process variable update formulas other than Equation 24. The rate integration module 180 may use first, second or higher-order methods including Euler methods, Runge-Kutta methods, backward-differentiating formulas, exponential integration techniques, or other suitable methods.

The iterative portion of the modeling process 100 may conclude when the process variable corresponding to the virtual time of the simulation indicates the end of the simulated time period. In implementation, new/updated values of the process variables computed by the modeling process 100 at each iteration of the time step 102 may be stored or saved for further computation or processing. For example, the values computed at different virtual times or different iterations of the virtual time step 102 may be combined to create a data set indicative of time-evolution of the process variables.

Upon completion of the iterative portion executed for a sequence of time steps 102, the modeling process 100 may include an additional module for computing one or more metrics of the computationally modeled bioreactor 120 based on the computed new values of the process variables. A metric of the one or more metrics may be a numeric value or a collection of numeric values in vectors, arrays, or other suitable data structures, a text value or a collection of text values in a suitable data structure, or a Boolean value or collection of Boolean values in a suitable data structure, The metric may indicate quality of the simulation and confidence in the output, or it may be a measure of performance of a hypothetical real bioreactor corresponding to the simulation. For example, the metric may be indicative of efficiency of converting substrate metabolites into the target product, with the efficiency being calculated in terms of financial cost and/or time. Additionally or alternatively, the metric may be indicative of the total amount of target product produced and/or product quality.

The modeling process 100 may include an additional module for generating, based on the metric of the modeled bioreactor, information displayed to a user via a user interface, a control setting for a real-world bioreactor, and/or a training set for an artificial intelligence model of a hypothetical real-world bioreactor similar in operation to the simulated bioreactor 120.

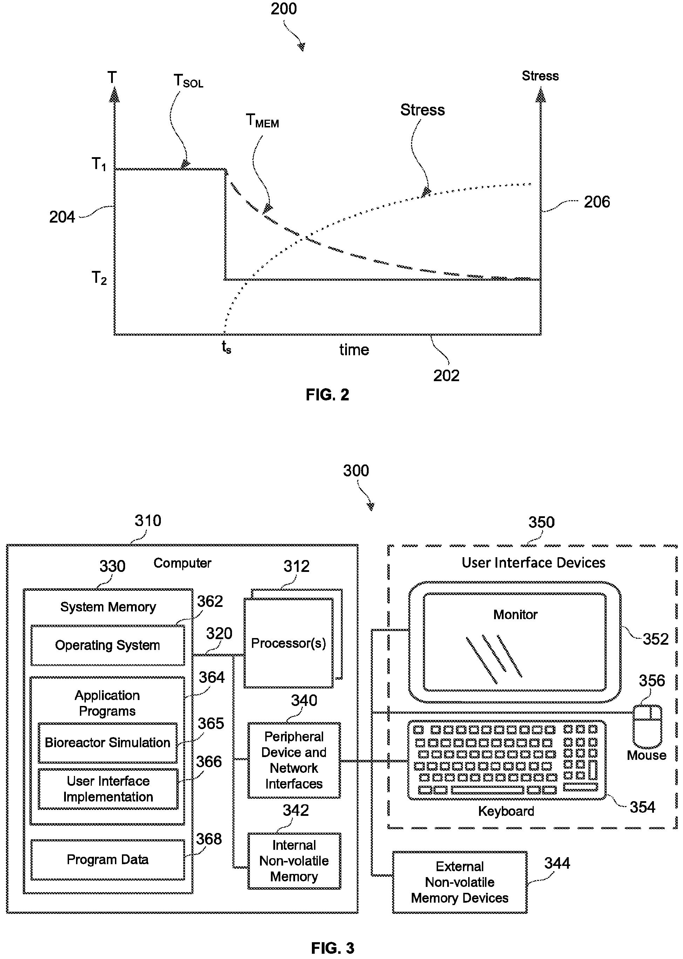

Figure 2 is a graph 200 depicting an exemplary effect of a temperature shift on the computed stress variable in an implementation of the modeling process 100. The graph 200 has a horizontal time axis 202 that represents time, increasing from left to right, as simulated by the modeling process 100. A vertical temperature axis 204 corresponds to a solid trace, labeled $T_{SOL}$, representing temperature of the extracellular solution 124 and a dashed trace, labeled $T_{MEM}$, representing memory temperature, while another vertical axis 206 corresponds to a dotted trace, labeled Stress, for representing the stress variable discussed above.

Referring still to Figure 2, a simulation implemented by the modeling process 100 may start with the process variable for the temperature $T_{SOL}$ of the extracellular solution 124 at a level $T_1$ that does not introduce substantial stress to the virtual biomass 130. The level $T_1$ may correspond to the optimal temperature for the growth of the cellular biomass 130. At a certain time, marked by $t_s$, the temperature of the solution 124 may decrease to a lower level $T_2$ that is less than the optimal temperature for the cellular biomass 130. The decrease in temperature may take place in response to one of the control variables 152, and may be abrupt, as shown in the graph 200, or may be more gradual. The abruptness of the temperature shift in $T_{SOL}$ may depend on the modeled control mechanism for the bioreactor 120, including the speed and quality of mixing of the modeled bioreactor contents. Even for the abrupt change in temperature shown in the graph 200, the memory temperature $T_{MEM}$ sensed by the biomass 130 may change more gradually, accounting for the biological response to a temperature shift and may follow the time-evolution represented by the following equation:

$$\frac{dT_{MEM}(t)}{dt} = k_{temp}(T_{SOL}(t) - T_{MEM}(t)) \quad \text{(Equation 26)},$$

where $k_{temp}$ is a model parameter that corresponds to the speed of adaptation of the cellular biomass 130 to a temperature shift. The rate integration module 180 may compute $T_{MEM}(t)$ by integrating Equation 25 and may compute *Stress* by numerically integrating the area between $T_{MEM}$ and $T_{SOL}$, using the equation

$$\frac{dStress(t)}{dt} = |T_{MEM}(t) - T_{SOL}(t)| \quad \text{(Equation 27)},$$

with the result represented by the dotted line on the graph 200 representation of *Stress*.

Figure 3 illustrates an exemplary computer system 300 that may implement the methods and techniques described herein. The computer system 300 of Figure 3 includes a computer 310. Components of the computer 310 may include, but are not limited to, one or more processors 312 in communicative connection, by way of a system bus 320, with a system memory 330. The one or more processors 312 may include one or more single-core or multi-core central processing units (CPUs), graphical processing units (GPUs), or any other suitable processor architecture. The system memory 330 may include a read-only memory (ROM) component and a random-access memory (RAM) component, for example. The RAM component of system memory 330 may be any of several types of RAM, including static RAM (SRAM), dynamic RAM (DRAM), synchronous dynamic RAM (SDRAM), double data rate SDRAM (DDR SDRAM), or another suitable type of RAM. The system bus 320 may include one or more types of bus structures including a memory bus or memory controller, a peripheral bus, or a local bus, etc., and may use any suitable bus architecture. By way of example, and not limitation, such architectures include the Industry Standard Architecture (ISA) bus, Micro Channel Architecture (MCA) bus, Enhanced ISA (EISA) bus, and Peripheral Component Interconnect (PCI) bus (also known as Mezzanine bus).

The one or more processors 312 also may be in communicative connection, by way of the system bus 320, with one or more peripheral device and network interfaces 340, and with an internal non-volatile memory 342. The internal non-volatile memory may be, for example, a hard disk drive (HDD) or a solid-state drive (SSD), for example. Additionally or alternatively, external non-volatile memory devices 344 may be in communicative connection with the computer 310 via the peripheral and network device interfaces 340. The peripheral device and network interfaces 340 may include a variety of connectors or adapters for communicatively connecting with devices outside the package or housing of the computer 310.

The peripheral device and network interfaces 340 may include one or more universal serial bus (USB) interfaces, one or more video connections, including but not limited to VGA (video graphics array), DVI (digital visual interface), and/or HDMI (high-definition multi-media interface). The peripheral device and network interfaces 340 may be connected to one or more user interface devices 350, including and not limited to a monitor 352, a keyboard 354, and a mouse 356. In some implementations, some or all of the user interface devices 350 are integral to the computer 310, and may be in communicative connection with the one or more processors 312 via the bus 320. Additionally or alternatively, the monitor 352, the keyboard 354, and the mouse 356 can be integrated as a touchscreen.

Additionally or alternatively to connecting to the user interface devices 350, the peripheral device interface 340 may communicatively connect to one or more external non-volatile memory devices 344. The non-volatile memory devices 344 may include HDDs, SSDs, as well as removable storage drives for optical, magnetic, or electronic media, including but not limited to compact disc (CD), digital versatile disc (DVD), magnetic tape, floppy disc, or flash memory. Additionally or alternatively, peripheral device and network interfaces 340 may communicatively connect to output devices including and not limited to printers, plotters, speaker, or any other suitable visual, auditory, tactile or haptic output devices.

The peripheral device and network interfaces 340 may include one or more network adapters to communicatively connect to one or more networks (not pictured) that may include a local-area network (LAN) and/or a wide-area network (WAN), such as the Internet. The connections may be wired connections or wireless connections, such as, for example, using radio or optical signals. By way of the connected network, the computing system 300 may communicatively connect to other computing systems, peripheral devices, or other devices.

Collectively, the one or more processors 312, the bus 320, the system memory 330, the peripheral device and network interfaces 340, the internal non-volatile memory 342, the external non-volatile memory devices 344, and the user interface devices 350 may be referred to as processing hardware of the computing system 300. The processing hardware that implements the modeling process 100 of Figure 1 may omit some of the elements depicted in Figure 3 or comprise additional elements.

In operation, the computing system 300 may load program instructions for execution by the one or more processors 312 into the system memory 330. The system may load the program instructions into the system memory 330 from the internal non-volatile memory 342 or from external non-volatile memory devices 344, for example. The program instructions loaded into the system memory may include an operating system 362, as well as a variety of application programs 364. In some implementations, the computing system 300 may cause other computing systems in communicative connections with the computing system 300 to load program instructions for execution by processors of the other computer systems.

The application programs 364 loaded into the system memory 330 of the computer 310 may include a bioreactor simulation program 365, containing instructions to implement, at least partially, the modeling process 100 of Figure 1 using the processing hardware of the computing system 300. In some implementations, other computing systems in communicative connection with the computing system 300 may implement portions of the modeling process 100 using processing hardware of the other computing systems. The application programs 364 loaded into the system memory 330 of the computer 310 may additionally include a user interface implementation program 366 that configures the hardware of the computing system 300 to implement portions of the modeling process 100 or to contribute to or enhance the implementation in a variety of ways. For example, the user interface implementation program 366 may configure the hardware to receive a plurality of the current values 150a of the process variables, the control variables 152, and/or input and output flow variables 182 at the beginning or, in some implementations, in the middle of the simulation. Additionally or alternatively, the user interface implementation program 366 may configure the hardware to display information to a user via a user interface rendered, for example, on the monitor 352.

The user interface implementation program 366 may configure the hardware to display information to a user in a variety of ways. In this context, to display information may mean to present it in visual form on the monitor 352, or on printed paper, or in audio form through speakers, or in tactile or haptic form through tactile or haptic devices or in a variety of other suitable ways. The user receiving the displayed information may be a scientist, a bioreactor operator, or any other person interested in the displayed information. In some implementations, the user is another machine capable of processing the displayed information and causing an action based the processed information. In some implementations and/or scenarios, a user receiving information through at least one of the user interface devices 350 and a user entering information through at least one of the user interface devices 350 are distinct users.

Returning to the application programs 364 that may include a bioreactor simulation program 365 and a user interface implementation program 366, the application programs 364, in operation, may access and alter a program data 368 portion of the system memory 330. The program data 368 may include the data received at the beginning of the simulation along with all the new data computed throughout the course of the simulation. The program data 368 may include, for example, all or some of the instances of the current values 150a of the process variables, the updated values 150b of the process variables, the control variables 152, and/or input and output flow variables 182. The program data 368 may include the index or value of the time step 102, along with other time parameters of the simulation. The other time parameters may include, for example, virtual start time, virtual end time and a variety of virtual transition times such as $t_s$ (i.e., the temperature shift time in Figure 2). Additionally, the program data 368 may include the one or more metrics of the computationally modeled bioreactor 120 based on the computed new values of the process variables and the generated output information based on the one or more metrics. The generated output information may include information intended for display to a user via a user interface rendered in one or more of the user interface devices 350 or a training set for an artificial intelligence model of a bioreactor intended, for example, for storage in internal non-volatile memory 342 or external non-volatile memory devices 344.

In some implementations, the bioreactor simulation program 365 and the user interface implementation program 366 are parts of a single application. In other implementations, the bioreactor simulation program 365 and the user interface implementation program 366 are separate applications sharing the hardware resources of the computer 310 simultaneously, or sequentially. For example, the user interface implementation program 366 may render a user interface on the user interface devices 350 to receive the data necessary for the bioreactor simulation program 365, and store the data in the internal non-volatile memory 342 or the external non-volatile memory devices 344. At a different time, the bioreactor simulation program 365 may use the data stored by the user interface implementation program 366 (or by another application) to implement the bioreactor modeling process 100, and to store any generated metrics or other output data in the internal non-volatile memory 342 or the external non-volatile memory devices 344. At a subsequent time, the user interface implementation program 366 may retrieve the output of the bioreactor simulation program 365 and generate appropriate output for the user.

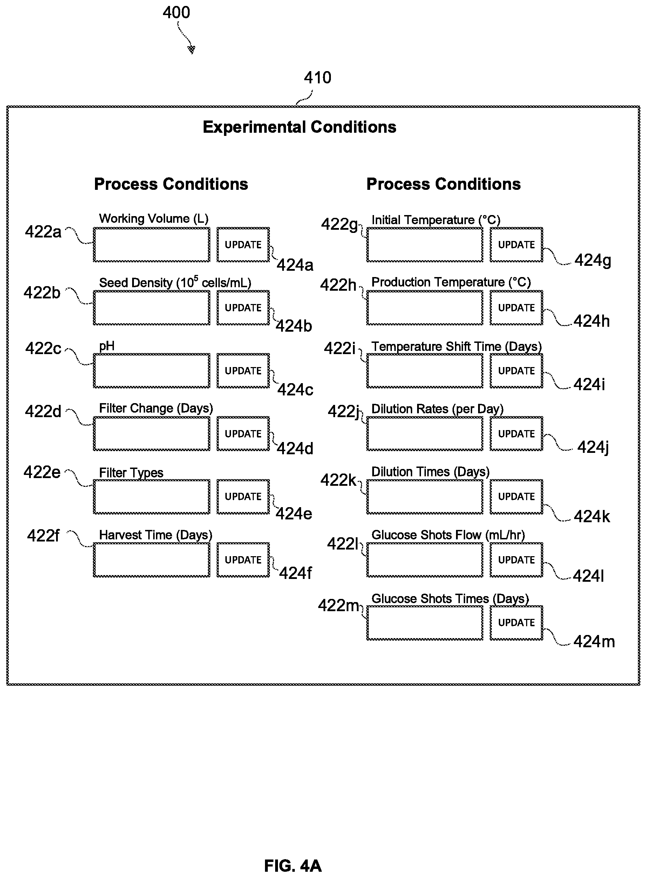

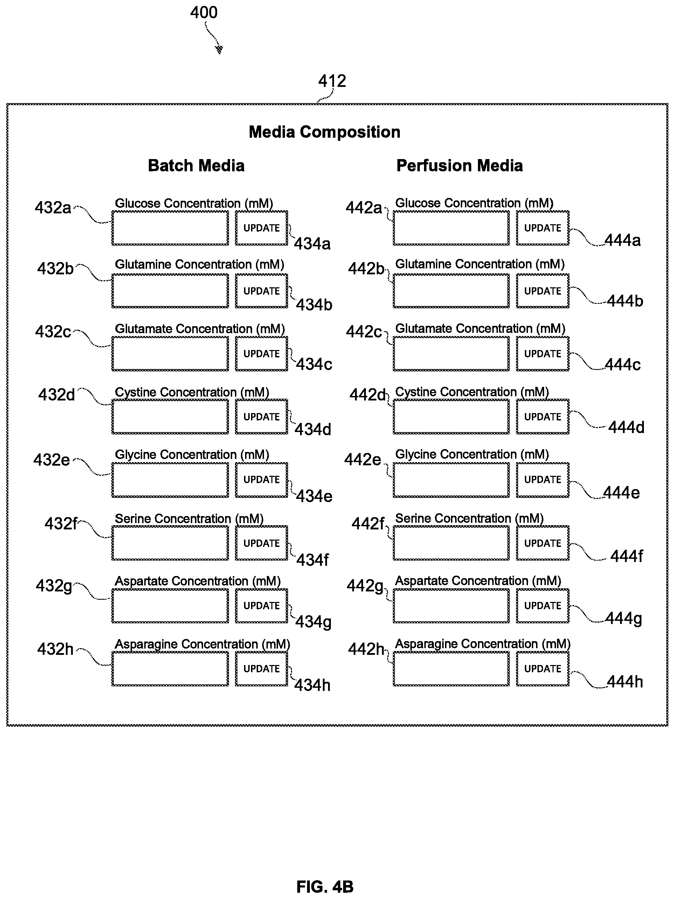

Figures 4A and 4B illustrate two panels of an exemplary user interface 400, which may be rendered on the monitor 352 by the user interface implementation program 366 running on the computer 310, for example. The user interface 400 may enable the modeling process (e.g., modeling process 100 of Figure 1) to receive at least some of the initial values of the process variables (e.g., the current values 150a for the first time step 102). To this end, the exemplary user interface 400 may include one panel 410 for receiving experimental condition variables from the user, and another panel 412 for receiving media composition variables from the user, with the user entering the information via the user interface devices 350 (e.g., the keyboard 354 and the mouse 356). The experimental conditions panel 410 in Figure 4A may contain input cells 422a-m where the user can enter numerical values for the designated initial values of the process variables and buttons 424a-m that the user can activate by clicking a button on the mouse 356 to cause the corresponding values to be loaded into program data 368.

Some of the values entered in the experimental conditions panel 410 may set the values of the control variables 152 in the modeling process 100. For example, filter change 422d, harvest time 422f, and glucose shots times 422m may indicate the virtual times within the simulated time interval when process variables may change based on the virtual controls, rather than by updating the process variables by integrating the computed rates of change.

The media composition panel 412 in Figure 4B enables the modeling process 100 to receive from the user, through the input cells 432a-h, initial process variable values indicative of metabolite concentrations in the batch media or the extracellular solution 124 of the batch mode of the bioreactor 120, and/or, through the input cells 442a-h, metabolite concentrations in the input stream 140 during the perfusion mode of operation of the bioreactor 120. Once the simulation time interval corresponding to the perfusion mode commences, the concentrations of metabolites in the input stream 140 may start affecting the process variables corresponding to the metabolite concentrations in the extracellular solution 124 by altering the corresponding rates of change computed by the rate integration module 180.

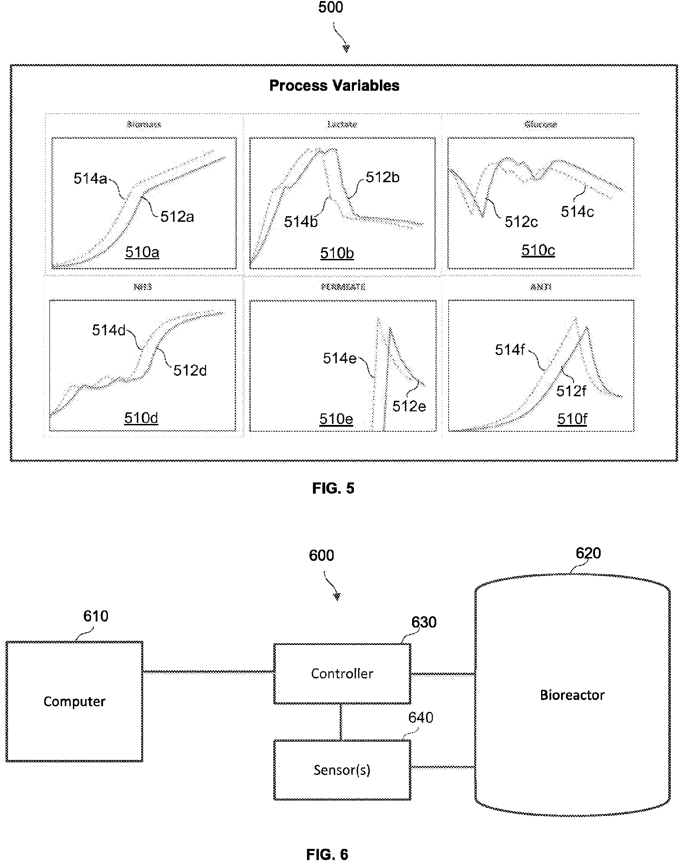

Figure 5 illustrates an exemplary user interface 500 that displays information to the user. The user interface implementation program 366 of Figure 3 may render the user interface 500 on the monitor 352, for example. The user interface implementation program 366 may generate the information needed to display the user interface 500 based on metrics computed by the bioreactor simulation program 365, and display the information in panels 510a-e. The displayed metrics may include traces 512a-f representing values of several process variables during the simulated time period. A user may select, through another user interface provided by the user interface implementation program 366, which variables to display. Additionally or alternatively, the user may choose to display another set of traces 514a-f representing values from a previously simulated time period. The traces 512a-f, 514a-f from the two simulations may reflect different process conditions, which may have been entered by the user, for example, through the user interface 400.

In one example, the panels 510a-e of the user interface 500 display, respectively, a trace 512a of the biomass variable, a trace 512b of the lactate variable, a trace 512c of the glucose variable, a trace 512d of the ammonia variable, a trace 512e of the permeate variable, and a trace 512f of the antibody variable. The comparison offered by the corresponding 514a-f traces from a previous simulation may provide the user with useful information about expected changes in a hypothetical bioreactor run performance, without having to conduct an *in vivo* experiment with a real-world bioreactor. The permeate traces 512e, 514e may indicate the virtual time evolution, for example over the course of a number of days, of the concentration or amount of the antibody product harvested from the output stream 142. The abrupt increases from the zero baseline of the permeate traces 512e, 514e may correspond to initiating virtual harvesting at the virtual times entered into the input cell 422f for harvest time of the user interface 400A. The initiation of harvesting also may correspond to a change from batch mode to perfusion mode of operation of the simulated bioreactor 120, or a change in the virtual filter 144 that controls the amount of the target antibody product in the output stream 142, and a corresponding change in the computation of updates to the antibody concentration variable by the rate integration module 180. Consequently, as the simulation of harvesting begins, the traces 512f, 514f indicative of antibody concentration in the extracellular solution begin to decrease.

The traces 512a, 514a for the biomass may indicate two distinct regions: a high rate of growth region followed by the slower growth region. The transition between the two regions may correspond to the introduction of stress, for example, by dropping temperature of the extracellular solution from $T_1$ to $T_2$ at the time $t_s$, as shown in Figure 2, and based on the values entered in the input cells 422g-i of the user interface 400A. The changes corresponding to the temperature shift in the metabolic byproduct traces 512b, 514b, 512d, 514d for lactate and ammonia may indicate a decrease in lactate production and an increase in ammonia production, as computed by the flux balance analysis 170 of the modeling process 100. The variability in the first half of the glucose traces 512c, 514c may correspond to the introduction of glucose shots into the input flow stream 140, according to the values entered in the input cells 422l-m of the user interface 400A. In the exemplary implementation, traces presented in the panels 510a-f imply mutual dependence of process variable values computed using the modeling process 100 by application programs 365, 366 running on the computing system 300.

Figure 6 illustrates a bioreactor control system 600, in an implementation where the program(s) for modeling a bioreactor (*e.g.,* bioreactor simulation program 365 and/or another application) cause the processing hardware of the computer 610 to generate a control setting for a real-world bioreactor 620. The computer may be the computer 310 in Figure 3, and may communicate the control setting over one of the interfaces of peripheral and network interfaces 340, for example. The bioreactor control system 600 may include, in addition to the real-world bioreactor 620, a controller 630, and one or more sensors 640. The controller 630 may be in communicative connection with the one or more sensors 640 that sense, measure, and/or transduce physical, chemical, and biological properties of the bioreactor 620 and any connected systems. The sensors 640 may measure the weight of the bioreactor 620, a liquid level within the bioreactor 620, temperature at a plurality of locations, acidity, turbidity, optical density, osmolarity, oxygen levels, concentrations of a variety of metabolites, chemical compositions of solid liquid or gas samples, cell counts, and any other suitable indicators of performance or operation of the bioreactor 620 and/or components fluidically or mechanically connected to the bioreactor 620, for example.

The controller 630 may control, through a communicative, electrical, or mechanical connection(s), a variety of mechanical and/or electrical components, including one or more heaters or heat exchangers, one or more mixers, transducers, pumps, valves and/or other devices for altering physical, chemical, and biological properties of the bioreactor 620 and its contents. In some implementations, the controller 630 controls one or more quantities associated with the bioreactor 620 (*e.g.*, temperature, glucose concentration, etc.) using respective proportional-integral-derivative (PID) controller hardware, firmware, and/or software. The controller, for example, may cause a change in the input flow rate or composition of the flow into the bioreactor 620, and/or in the temperature of bioreactor 620 contents. The controller may initiate changes in the modes of operation of the bioreactor 620, including, for example, the switch from batch operation mode to continuous flow operation to perfusion mode, from high growth rate mode in the substantially optimal environment for cellular growth to the stressed mode for increasing production of a target product, from the production mode to harvesting mode, and/or any other desired changes. The controller 630 may take any of the actions described above in response to one or more control settings communicated to the controller 630 by the computer 610. Additionally or alternatively, the controller 630 may communicate the signals from the sensors 640 to the computer 610. The computer 610 may use the signals from the sensors 640 in implementing the modeling process 100. For example, the computer 610 may adjust one or more modeling parameters of the model 104 based at least in part on the sensor signals received from the controller 630. In some implementations, the sensors 640 are in direct communicative connection with the computer 610. In some implementations where the computer 610 is the computer 310 of Figure 3, a user may enter values indicative of sensor signals through user interface devices 350 or through external non-volatile memory devices 344. In some implementations, the controller 630 implements model predictive control (MFC) to control the mechanical and/or electrical components.

Whether or not the signals from the sensors 640 are used in implementing the modeling process 100, a control setting generated by the computer 610 and based at least in part on a metric of the computationally modeled bioreactor 120 may, by way of the controller 630, alter the operation of the real-world bioreactor 620. For example, the modeling process 100 implemented on the computer 610 may compute a metric indicative of an optimal time to change an operation mode and send the corresponding control setting to the controller 630. In some implementations, the computer 310 and computer 610 are different devices in communicative connection with each other, and the control setting is generated by the computer 310. The control setting may be transferred to the computer 610 by way of a network through one of the peripheral device and network interfaces 340, or by way of one of the external non-volatile memory devices 344, for example. In some implementations, the control settings for the real-world bioreactor 620 are part of real-time model-based control of the bioreactor 620. In other implementations, the control-setting is based on a model-generated metric at a prior time as a part of an *in silico* experiment relevant to the operation of the real-world bioreactor 620.

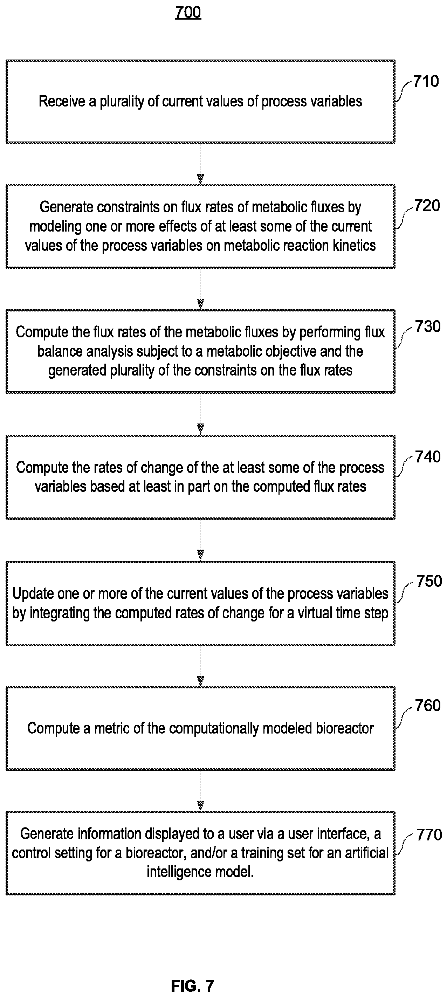

Figure 7 is a flow chart depicting a computer-implemented method 700 of computationally modeling a bioreactor, according to one example implementation. The method 700 may apply the computational model 104 of Figure 1, and may include execution of the modeling process 100 of Figure 1, for example. Moreover, the method 700 may be implemented by the processor(s) 312 of the computer 310 (*e.g.,* when executing the bioreactor simulation program 365), or by another suitable computing device or computing system. The method 700 may be adapted to model spatially inhomogeneous bioreactors using, for example, the techniques described above.

The method 700 includes receiving a plurality of current values of process variables (block 710). The values may be received from a user via a graphical user interface, as exemplified in Figures 4A and 4B and the associated discussion. As illustrated in Figure 6 and the associated discussion, in some implementations the received values may be based on measurements of a real bioreactor. Additional or alternative ways to receive or obtain the current values of the process variables may include loading values from computer memory (*e.g.,* previously computed values) and/or loading predetermined default values from computer storage.

The process variables received at block 710 may describe virtual contents of a bioreactor, such as the virtual cellular biomass 130 in the virtual extracellular solution 124 of the modeled bioreactor 120 of Figure 1, for example. The process variables may include temperature, acidity, and/or one or more variables indicative of total osmolarity, for example, describing an extracellular solution (*e.g.,* extracellular solution 124), biomass (*e.g.,* biomass 130), and/or input and output streams (*e.g.,* input stream 140 and/or output stream 142). Additionally or alternatively, the process variables may indicate the amount or concentration of virtual biomass (*e.g.,* biomass 130) and/or the amount or concentration of any of the metabolites in a virtual extracellular solution (*e.g.,* solution 124), and/or input and output streams (*e.g.,* input stream 140 and/or output stream 142). The metabolites with amounts or concentrations described by the process variables may include oxygen, carbon dioxide, ammonia, lactate, glucose, asparagine, glutamine, glycine, and/or any other suitable metabolite. The variables may describe extracellular and/or intracellular (inside the virtual cells) amounts or concentrations of metabolites. In some implementations, the process variables describe metabolite concentrations in different cellular organelles or apparatus. Furthermore, when the method 700 models inhomogeneous bioreactors, the process variables may describe each of the modeled bioreactor portions.

To compute new values of the process variables during a simulated time period, the method 700 may generate, at block 720, for each given time step (*e.g.,* multiple iterations of time step 102), a plurality of constraints on flux rates of metabolic fluxes (*e.g.,* describing the virtual cellular biomass 130). The metabolic fluxes may be the fluxes depicted as 132a-i of Figure 1, the fluxes listed in Table 2, or any other suitable description of metabolic pathways and/or biochemical reactions in the cellular metabolic process. The metabolic fluxes may describe metabolic networks of different types of cells, including bacterial cells, animal cells, and/or fungal cells. The types of cells modeled by the cellular biomass may include stem cells or reproductive cells, such as, for example, Chinese hamster ovary (CHO) cells.

The method 700 may generate the constraints on flux rates by modeling one or more effects of at least some of the current values (*e.g.,* the current values 150a) of the process variables on metabolic reaction kinetics using a kinetics model such as, for example, the kinetics model 160. The constrained flux rates may include glucose uptake, glutamine uptake, asparagine uptake, oxygen uptake, and/or any other flux rate directly or indirectly limited, on the high end and/or on the low end, by extracellular conditions (*e.g.,* the conditions of the extracellular solution 124). The limiting conditions may include temperature, acidity, and/or osmolarity, for example. The constraints on the metabolic flux rates may include upper and/or lower limits on the flux rates for biomass growth or energy consumption for cellular maintenance. Modeling the one or more effects of the current values of the process variables on the metabolic reaction kinetics may include computing the effect of temperature, acidity, and/or osmolarity on the upper limit of the metabolite uptake rate by multiplying an upper limit under ideal conditions with a correction factor indicative of a reduction in the uptake rate due to non-ideal conditions. The correction factors may be constructed in view of experimental observation of real-world cellular cultures affected by changes in conditions. These and other experimental observations may be included in a calibration procedure for a computational model, such as the computational model 104.

The effects of the current values of the process variables on metabolic reaction kinetics may include the effect of stress on the lower limit of energy consumption rate for cellular maintenance. Stress may be increased to induce a higher rate of production of a target product. In one implementation, as illustrated in Figure 2, a temperature shift induces stress, requiring the cellular biomass to expand more energy on non-growth processes. Other ways to induce stress in the cellular biomass may be to change acidity or osmolarity of the extracellular solution. The effects of the current values of the process variables on metabolic reaction kinetics may include effects of metabolic byproducts. Lactate concentration and ammonia concentration may be among the factors that affect constraints on the flux rates computed by the kinetics model. Modeling the effects above may include calibrating the modeled effects with experimental data from a real-world cell culture.

The method 700 may continue, at block 730, with computing the flux rates of the metabolic fluxes (*e.g.,* for the virtual biomass 130), by performing flux balance analysis (*e.g.,* flux balance analysis 170). The flux balance analysis may be subject to a metabolic objective (*e.g.,* the metabolic objective 172) and the generated plurality of the constraints (*e.g.,* constraints 164) on the flux rates. Some exemplary metabolic objectives are listed above in Table 4. A broad class of metabolic objectives may be generated by minimizing a ratio of linear combinations of at least some of the flux rates and squares of flux rates. Each weight in the linear combinations may be positive, negative, or zero. Thus, the generated metabolic objective may, for example, effectively maximize growth per total flux ratio, or maximize growth relative to substrate metabolite consumption. In a scenario with insufficient maintenance energy, for example, non-negative growth may be impossible to achieve, and maximizing the maintenance energy may be the metabolic objective used in the flux balance analysis. When there is sufficient maintenance energy, maximizing the rate of growth of the virtual biomass may be the metabolic objective used in the flux balance analysis.

At block 740, the method 700 may compute the rates of change of the at least some of the process variables based at least in part on the flux rates computed at block 730. Computing the rates of change may include receiving one or more variables indicative of flow rate and composition of the input stream (*e.g.,* the flow variables 182 corresponding to the input stream 140), the output stream (*e.g.,* the flow variables 182 corresponding to the output stream 142), and/or the output filter (*e.g.,* filter 144).

The method 700 may then update, at block 750, one or more of the current values (e.g., current values 150a) of the process variables, at least in part by integrating one or more of the computed rates of change for the duration of the virtual time step. This may conclude an iteration of a given virtual time step. For example, the updated values at the conclusion of one time step may become the current values used for the following time step. Block 750 may be performed by the rate integration module 180 of Figure 1, for example, when computing the updated values 150b from the current values 150a. The method 700 may compute (using, for example, the rate integration module 180) the updated values at least in part by accounting for the variables indicative of flow rate and composition of input and/or output streams. The method 700 may include, at block 750, the stream variables in the mass-balance difference or differential equations discussed above, and solve the equations by integration. The mass-balance equations may, additionally or alternatively, include the effects of the variables indicative of properties of the virtual output filter on the composition of the virtual output stream.

When the method 700 models an inhomogeneous bioreactor, the method may include updating the process variables for each portion of the modeled bioreactor at least in part based on a computed velocity field. The velocity field may be computed, for example, using a CFD model. The method 700 may update the process variables for a portion of the bioreactor in view of the process variables for the neighboring portions of the bioreactor. In some implementations, the method 700 may define gradients for at least some of the process variables in at least at some of the modeled portions of the bioreactor.

Before moving on to block 760, the method 700 may iterate blocks 710-750 for a part or an entirety of the simulated time period, divided into the sequence of time steps. In other implementations, block 760 occurs in parallel with the iterations associated with one or more of blocks 720-750. At block 760, the new values of the process variables computed during any virtual time step or steps are used to compute a metric of the modeled bioreactor or, more generally, a model of the system containing the virtual bioreactor (*e.g.,* model 104). The metric of the computationally modeled bioreactor may indicate effectiveness or efficiency of the computationally modeled bioreactor in producing a target product. For example, the metric may indicate a total product output, a yield with respect to energy and/or substrate metabolites, productivity of the reactor, and/or quality of the target product. In some implementations, multiple metrics are computed at block 760. The metric(s), additionally or alternatively, may include values of at least one of the process variables at different virtual times within the simulated time period, as illustrated, for example, in Figure 5 and the associated description. For an inhomogeneous bioreactor, metrics computed for portions of the bioreactor may contribute to the metric for the bioreactor as a whole. In some implementations, however, the bioreactor metric may be based on one or more portions of the bioreactor that include the output stream out of the bioreactor. Additionally or alternatively, the method 700 may generate, at block 760, a metric indicative of inhomogeneity within the bioreactor.

At block 770 of the method 700, information may be generated and displayed to a user via a user interface rendered, for example, on a monitor (*e.g.,* the monitor 352). The method 700 may generate the displayed information based at least in part on the metric computed at block 760. In some implementations, block 770 additionally or alternatively includes generating a control setting for a real-world bioreactor (*e.g.* bioreactor 620), as depicted, for example, in Figure 6 and the accompanying description. In some implementations, the method 700 is integrated into a model-based control scheme of a real-world bioreactor (*e.g.,* using model predictive control). When modeling inhomogeneous bioreactors, the method 700 may display inhomogeneity at the user interface, or generate one or more control settings based on, for example, on a computed metric of inhomogeneity. For example, mixing may be initiated or enhanced when the metric indicative of inhomogeneity exceeds a threshold.

Additionally or alternatively, block 770 may include generating a training set for an artificial intelligence model of a bioreactor by using the metric computed at block 760 as a ground truth label for a bioreactor model, with the model being described by a set of parameters. A plurality of executions of the method 700 may allow an accumulation of a plurality of data sets of model parameters and corresponding metrics. The datasets may be used to train the artificial intelligence model based on neural networks, convolutional neural networks, decision trees, clustering algorithms, and/or other suitable machine learning techniques. The trained artificial intelligence model may then estimate or predict a new metric based on the bioreactor model parameters, without needing to again execute the computational modeling method 700.

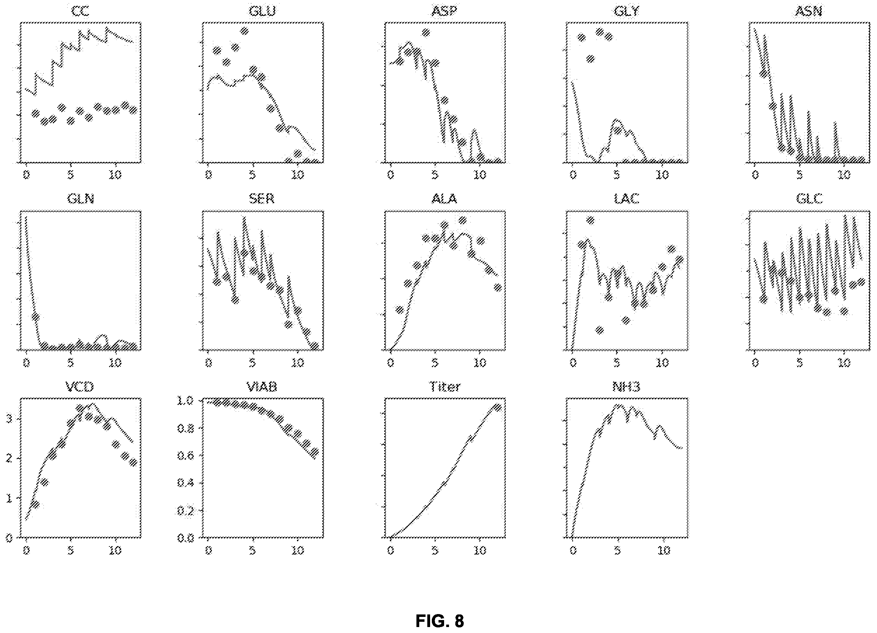

Fig. 8 illustrates an example evolution of process variables and metrics computed using the method 700. A set of data for initial process variables (including pH, temperature, media compositions) and a media feed schedule (input stream) for a manufacturing process of a monoclonal antibody were received. The process was computationally modeled according to the workflow illustrated in Figs. 1 and 7 and described above. Values of process variables (in arbitrary units) corresponding to metabolite concentrations (CC-GLC, as defined in Table 3), and metrics of cell viability, viable cell density (VCD), and antibody product (titer) calculated by the model are plotted as solid lines in Fig. 8. The values of metabolite concentrations, viable cell density (VCD), cell viability, and antibody titer measured during a real-world execution of the same manufacturing process are plotted as dots on the same scale as corresponding computed values. The computational models substantially predicted the observed real-world process performance. The sharp variations in concentrations of certain metabolites were due to the discontinuous feed of the bioreactor - metabolites were added as batches at a certain time of day. The measured metabolites lack the sharp variations because the measurements were limited to the time of day prior to the addition of feed metabolites. The metric of viability (VIAB) represents the fraction cells in the biomass still capable of producing product and depends on the accumulated effect of cell death, as described above. The VCD then represents biomass concentration multiplied by viability.

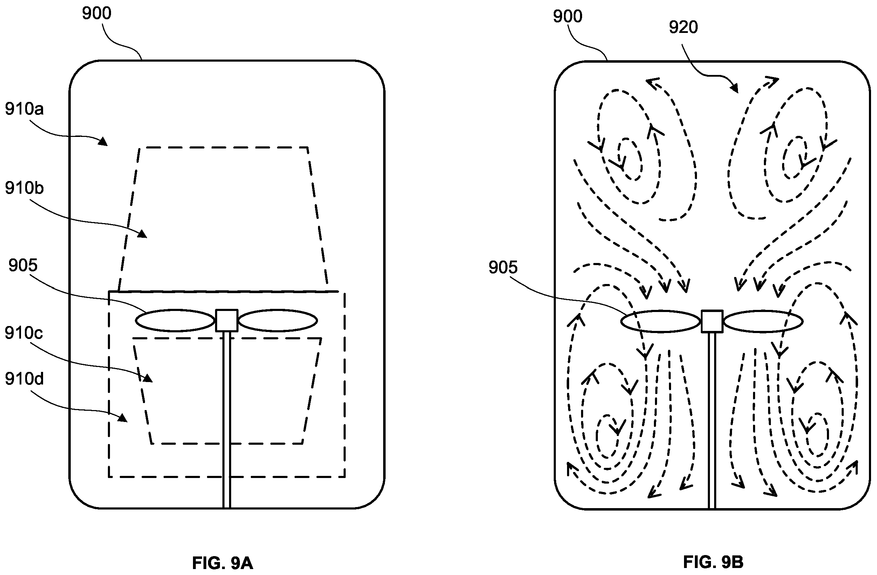

Figs. 9A and 9B schematically illustrate an example approach to modeling spatial inhomogeneity in a bioreactor 900. The bioreactor 900 may include an impeller 905 or another mixing device configured to mix bioreactor contents. Fig. 9A illustrates an example partitioning of the bioreactor 900 into multiple portions 910a-d. A homogeneous metabolic model (e.g., model 104) may represent each of the portions 910a-d. Each of the portions 910a-d may share a border with one or more other portions. In the illustrated example, the portion 910c is entirely disposed within the portion 910d, while the portion 910d borders all three other portions, i.e. 910a-c. Inlets and outlets (not illustrated) into the bioreactor 900 may add or remove metabolites to or from any of the modeled portions 910a-c. For each of the portions 910a-d, the modeled input and output streams (e.g., streams 140, 142) may include flow of virtual contents due to the inlets and outlets as well as across the borders with the neighboring portions. The flow of virtual contents across the borders between the bioreactor portions 910a-d may follow an example velocity field 920 illustrated in Fig. 9B. Computing the input and output streams may include estimating gradients of process variables at the borders as corresponding differences in process variable values in the bordering regions, integrating the velocity field 920 at each border (and normal to the border), and multiplying the estimated gradients by the integrated values of the velocity field 920 corresponding to each border.

Fig. 9B illustrates the velocity field 920 as a set of dashed arrows. A CFD model may compute the velocity field 920 based on a volume discretization of the bioreactor 900 and suitable computational techniques. In some implementations, a method (e.g., method 700) of computationally modeling the bioreactor 900 may determine the velocity field 920 based at least in part on measurements (e.g., particle velocimetry) or a combination of pre-computed or measured velocity fields. The velocity field 920 may depend of the geometry of the bioreactor 900 and the impeller 905, as well as on the speed of the impeller 905. The velocity field 920 may also depend on input and output streams of any inlets and outlets of the bioreactor 900. Importantly, under a static set of conditions, the velocity field 920 may have a steady state from which the model may compute the flow of virtual contents across the borders between the bioreactor portions 910a-d. Additionally or alternatively, coupled fluid dynamics and metabolic models may account for dynamic changes in the velocity field 920 and the corresponding effect on evolution of metabolite concentrations in the bioreactor portions 910a-d.

The following list of options reflects a variety of the embodiments explicitly contemplated by the present disclosure.

Option 1. A method of computationally modeling a bioreactor, wherein the method comprises:

receiving a plurality of current values of process variables, the process variables describing virtual contents of at least a portion of the modeled bioreactor, and the virtual contents including virtual cellular biomass in a virtual extracellular solution;

computing, by processing hardware of a computing system, new values of the process variables during a simulated time period, at least in part by

generating a plurality of constraints on flux rates of metabolic fluxes describing the virtual cellular biomass by modeling one or more effects of at least some of the current values of the process variables on metabolic reaction kinetics,

computing the flux rates of the metabolic fluxes by performing flux balance analysis subject to a metabolic objective and the generated plurality of the constraints on the flux rates,

computing rates of change of at least some of the process variables based at least in part on the computed flux rates, and

updating one or more of the current values of the process variables at least in part by integrating one or more of the computed rates of change for a virtual time step within the simulated time period;

computing, by the processing hardware, a metric of the computationally modeled bioreactor based at least in part on the computed new values of the process variables; and

generating, by the processing hardware, and based on the metric of the modeled bioreactor one or more of i) information displayed to a user via a user interface, ii) a control setting for a real-world bioreactor, or iii) a training set for an artificial intelligence model of a bioreactor.

Option 2. The method of option 1, wherein the process variables include at least one of: temperature, acidity or one or more variables indicative of total osmolarity of the virtual contents.

Option 3. The method of any one of options 1-2, wherein the process variables include one or more variables indicative of concentration of the virtual cellular biomass and one or more variables indicative of extracellular metabolite concentrations in the virtual extracellular solution.

Option 4. The method of option 3, wherein the one or more variables indicative of the extracellular metabolite concentrations in the virtual extracellular solution include one or more variables indicative of concentrations of at least one of: oxygen, carbon dioxide, or ammonia.

Option 5. The method of any one of options 3-4, wherein the one or more variables indicative of the extracellular metabolite concentrations in the virtual extracellular solution include variables indicative of concentrations of one or more of glucose, asparagine, glutamine, or glycine.

Option 6. The method of any one of options 3-5, wherein the one or more variables indicative of the extracellular metabolite concentrations in the virtual extracellular solution include variables indicative of concentration of at least one target metabolic product.

Option 7. The method of any one of options 3-6, wherein the process variables additionally include one or more variables indicative of one or more intracellular metabolite concentrations in the virtual cellular biomass.

Option 8. The method of any one of options 1-7, wherein the plurality of the constraints on the flux rates includes an upper limit on at least one of i) glucose uptake, ii) glutamine uptake, iii) asparagine uptake, or iv) oxygen uptake.

Option 9. The method of any one of options 1-8, wherein the plurality of the constraints on the rates of change includes a lower limit on an energy consumption rate for cellular maintenance.

Option 10. The method of any one of options 1-9, wherein the one or more effects of at least some of the current values of the process variables on the metabolic reaction kinetics includes an effect of a value of at least one of: i) temperature, ii) acidity, or iii) osmolarity on an upper limit of a metabolite uptake rate.

Option 11. The method of option 10, wherein modeling the one or more effects of at least some of the current values of the process variables on the metabolic reaction kinetics includes computing the effect of the value of at least one of: i) temperature, ii) acidity, or iii) osmolarity on the upper limit of the metabolite uptake rate by multiplying the upper limit with a correction factor indicative of a reduction in the uptake rate due to non-ideal conditions.

Option 12. The method of any one of options 1-11, wherein the one or more effects of the at least some of the current values of the process variables on metabolic reaction kinetics includes an effect of stress on a lower limit of energy consumption rate for cellular maintenance.

Option 13. The method of option 12, wherein modeling the effect of stress includes computing the effect based at least in part on a cumulative effect of a temperature shift on the virtual cellular biomass.

Option 14. The method of any one of options 1-13, wherein the one or more effects of the at least some of the current values of the process variables on metabolic reaction kinetics include an effect of concentration of at least one of metabolic byproducts.

Option 15. The method of any one of options 1-14, wherein modeling the one or more effects includes calibrating the modeled effects with experimental data from a real-world cell culture.

Option 16. The method of any one of options 1-15, wherein the metabolic objective includes minimizing a ratio of linear combinations of the at least some of the flux rates and squares of flux rates in the flux balance analysis.

Option 17. The method of any one of options 1-15, wherein the metabolic objective includes minimizing a linear combination of at least some of the flux rates in the flux balance analysis.

Option 18. The method of option 17, wherein the linear combination is a sum of the fluxes in the flux balance analysis.

Option 19. The method of any one of options 1-18, wherein the metabolic objective includes maximizing energy consumption rate for cellular maintenance.

Option 20. The method of options 1-19, wherein the metabolic objective includes maximizing a rate of growth of the virtual cellular biomass.

Option 21. The method of any one of options 1-20, further comprising:

receiving one or more variables indicative of flow rates and compositions of one or more virtual input streams into the modeled bioreactor, and wherein
updating the at least some of the current values of the process variables includes accounting for the flow rates and the compositions of the one or more virtual input streams.

Option 22. The method of option 21, further comprising:

receiving one or more variables indicative of properties of a virtual output filter;
computing compositions of one or more virtual output streams from the modeled bioreactor using the properties of the virtual output filter, and wherein
updating the at least some of the current values of the process variables includes accounting for the compositions of the one or more virtual output streams.

Option 23. The method of option 21, wherein updating at least some of the current values of the process variables includes solving mass-balance equations.

Option 24. The method of any one of options 1-23, further comprising:

receiving one or more control variables, and
updating an affected set of the current values of the process variables at least in part based on the received control variables.

Option 25. The method of any one of options 1-24, wherein
the metric of the modeled bioreactor includes values of at least one of the process variables at different virtual times within the simulated time period.

Option 26. The method of any one of options 1-25, wherein
the metric of the computationally modeled bioreactor indicates effectiveness or efficiency of the computationally modeled bioreactor in producing a target product.

Option 27. The method of any one of options 1-26, wherein

the method comprises generating the control setting, and wherein
the method further comprises controlling an input to a real-world bioreactor based on the generated control setting.

Option 28. The method of any one of options 1-27, wherein
the method comprises generating the training set for an artificial intelligence model of a bioreactor.

Option 29. The method of any one of options 1-28, wherein
the virtual cellular biomass comprises virtual Chinese hamster ovary (CHO) cells.

Option 30. The method of any one of options 1-29, wherein receiving the plurality of the current values includes at least one of

i) receiving values from a user via a graphical user interface;
ii) receiving values based on measurements of a real bioreactor;
iii) loading values from computer memory and based on previously computed values; or
iv) loading predetermined default values from computer storage.

Option 31. The method of any one of options 1-30, wherein the modeled bioreactor is a spatially homogeneous bioreactor, and the at least the portion of the modeled bioreactor is an entirety of the spatially homogeneous bioreactor.

Option 32. The method of any one of options 1-30, wherein:

the modeled bioreactor is a spatially heterogeneous bioreactor;
the at least the portion of the modeled bioreactor is a first portion of the modeled bioreactor;
the process variables are first process variables;
the method further includes receiving a plurality of current values of second process variables, the second process variables describing second virtual contents of a second portion of the modeled bioreactor; and
computing the new values of the first process variables during the simulated time period is based in part on the received plurality of the current values of the second process variables.

Option 33. The method of option 32, wherein computing the new values of the first process variables during the simulated time period includes computing a gradient of at least one of the first process variables based on a value of a corresponding one of the second process variables.

Option 34. The method of any one of options 32-33, further comprising:
determining one or more velocities associated with the virtual contents of the first portion of the modeled bioreactor.

Option 35. The method of option 34, wherein:
determining the one or more velocities is based at least in part on computational fluid dynamics.

Option 36. A non-transitory computer-readable medium storing instructions for computationally modeling a bioreactor, wherein the instructions, when executed by one or more processors, cause the one or more processors to:

receive a plurality of current values of process variables, the process variables describing virtual contents of the modeled bioreactor, and the virtual contents including virtual cellular biomass in a virtual extracellular solution;
compute, by processing hardware of a computing system, new values of the process variables during a simulated time period, at least in part by

generating a plurality of constraints on flux rates of metabolic fluxes describing the virtual cellular biomass by modeling one or more effects of at least some of the current values of the process variables on metabolic reaction kinetics,

computing the flux rates of the metabolic fluxes by performing flux balance analysis subject to a metabolic objective and the generated plurality of the constraints on the flux rates,

computing rates of change of at least some of the process variables based at least in part on the computed flux rates, and

updating one or more of the current values of the process variables at least in part by integrating one or more of the computed rates of change for a virtual time step within the simulated time period;

compute, by the processing hardware, a metric of the modeled bioreactor based on the computed new values of the process variables; and

generate, by the processing hardware, and based on the metric of the modeled bioreactor one or more of i) information displayed to a user via a user interface, ii) a control setting for a real-world bioreactor, or iii) a training set for an artificial intelligence model of a bioreactor.

Option 37. The non-transitory computer-readable medium of option 36, wherein the process variables include at least one of: temperature, acidity or one or more variables indicative of total osmolarity of the virtual contents.

Option 38. The non-transitory computer-readable medium of any one of options 36-37, wherein the process variables include one or more variables indicative of concentration of the virtual cellular biomass and one or more variables indicative of extracellular metabolite concentrations in the virtual extracellular solution.

Option 39. The non-transitory computer-readable medium of option 38, wherein the one or more variables indicative of the extracellular metabolite concentrations in the virtual extracellular solution include one or more variables indicative of concentrations of at least one of: oxygen, carbon dioxide, or ammonia.

Option 40. The non-transitory computer-readable medium of any one of options 38-39, wherein the one or more variables indicative of the extracellular metabolite concentrations in the virtual extracellular solution include variables indicative of concentrations of one or more of glucose, asparagine, glutamine, or glycine.

Option 41. The non-transitory computer-readable medium of any one of options 38-40, wherein the one or more variables indicative of the extracellular metabolite concentrations in the virtual extracellular solution include variables indicative of concentration of at least one target metabolic product.

Option 42. The non-transitory computer-readable medium of any one of options 38-41, wherein the process variables additionally include variables indicative of one or more intracellular metabolite concentrations in the virtual cellular biomass.

Option 43. The non-transitory computer-readable medium of any one of options 36-42, wherein the plurality of the constraints on rates of change includes an upper limit on at least one of i) glucose uptake, ii) glutamine uptake, iii) asparagine uptake, or iv) oxygen uptake.

Option 44. The non-transitory computer-readable medium of any one of options 36-43, wherein the plurality of the constraints on rates of change includes a lower limit on an energy consumption rate for cellular maintenance.

Option 45. The non-transitory computer-readable medium of any one of options 36-44, wherein the one or more effects of at least some of the current values of the process variables on the metabolic reaction kinetics includes an effect of a value of at least one of:
i) temperature, ii) acidity, or iii) osmolarity on an upper limit of a metabolite uptake rate.

Option 46. The non-transitory computer-readable medium of option 45, wherein
modeling the one or more effects of at least some of the current values of the process variables on the metabolic reaction kinetics includes computing the effect of the value of at least one of: i) temperature, ii) acidity, or iii) osmolarity on the upper limit of the metabolite uptake rate by multiplying the upper limit with a correction factor indicative of a reduction in the uptake rate due to non-ideal conditions.

Option 47. The non-transitory computer-readable medium of any one of options 36-46, wherein the one or more effects of the at least some of the current values of the process variables on metabolic reaction kinetics include an effect of stress on a lower limit of energy consumption rate for cellular maintenance.

Option 48. The non-transitory computer-readable medium of option 47, wherein modeling the effect of stress includes computing the effect based at least in part on a cumulative effect of a temperature shift on the virtual cellular biomass.

Option 49. The non-transitory computer-readable medium of any one of options 36-48, wherein the one or more effects of the at least some of the current values of the process variables on metabolic reaction kinetics include an effect of concentration of at least one of metabolic byproducts.

Option 50. The non-transitory computer-readable medium of any one of options 36-49, wherein modeling the one or more effects include calibrating the modeled effects with experimental data from a real-world cell culture.

Option 51. The non-transitory computer-readable medium of any one of options 36-50, wherein the metabolic objective includes minimizing a linear combination of at least some of the rates of change.

Option 52. The non-transitory computer-readable medium of any one of options 36-51, wherein the metabolic objective includes minimizing a linear combination of at least some fluxes in the flux balance analysis.

Option 53. The non-transitory computer-readable medium of option 52, wherein the linear combination is a sum of the fluxes in the flux balance analysis.

Option 54. The non-transitory computer-readable medium of any one of options 36-53, wherein the metabolic objective includes maximizing energy consumption rate for cellular maintenance.

Option 55. The non-transitory computer-readable medium of any one of options 36-54, wherein the metabolic objective includes maximizing a rate of growth of the virtual cellular biomass.

Option 56. The non-transitory computer-readable medium of any one of options 36-55, wherein the instructions further cause the one or more processors to:

> receive one or more variables indicative of flow rates and compositions of one or more virtual input streams into the modeled bioreactor, and wherein
> updating the at least some of the current values of the process variables includes accounting for the flow rates and the compositions of the one or more virtual input streams.

Option 57. The non-transitory computer-readable medium of option 56, wherein the instructions further cause the one or more processors to:

> receive one or more variables indicative of properties of a virtual output filter;
> compute compositions of one or more virtual output streams from the modeled bioreactor using the properties of the virtual output filter, and wherein
> updating the at least some of the current values of the process variables includes accounting for the compositions of the one or more virtual output streams.

Option 58. The non-transitory computer-readable medium of options 56-57, wherein updating the at least some of the current values of the process variables includes solving mass-balance equations.

Option 59. The non-transitory computer-readable medium of any one of options 36-58, wherein the instructions further cause the one or more processors to:

> receive one or more control variables, and
> update an affected set of the current values of the process variables at least in part based on the received control variables.

Option 60. The non-transitory computer-readable medium of any one of options 36-59, wherein

the metric of the modeled bioreactor includes values of at least one of the process variables at different virtual times within the simulated time period.

Option 61. The non-transitory computer-readable medium of any one of options 36-60, wherein

the metric of the modeled bioreactor indicates effectiveness or efficiency of the modeled bioreactor in producing a target product.

Option 62. The non-transitory computer-readable medium of any one of options 36-61, wherein the instructions

cause the one or more processors to generate the control setting, and
further cause the one or more processors to control an input to a real-world bioreactor based on the generated control setting.

Option 63. The non-transitory computer-readable medium of any one of options 36-62, wherein the instructions cause the one or more processors to generate the training set for an artificial intelligence model of a bioreactor.

Option 64. The non-transitory computer-readable medium of any one of options 36-63, wherein the virtual cellular biomass comprises virtual Chinese hamster ovary (CHO) cells.

Option 65. The non-transitory computer-readable medium of any one of options 36-64, wherein receiving the plurality of the current values includes at least one of:

i) receiving values from a user via a graphical user interface;
ii) receiving values based on measurements of a real bioreactor;
iii) loading values from computer memory and based on previously computed values; or
iv) loading predetermined default values from computer storage.

Option 66. The non-transitory computer-readable medium of any of the options 36-65, wherein the modeled bioreactor is a spatially homogeneous bioreactor, and the at least the portion of the modeled bioreactor is an entirety of the spatially homogeneous bioreactor.

Option 67. The non-transitory computer-readable medium of any of the options 36-65, wherein:

the modeled bioreactor is a spatially heterogeneous bioreactor;
the at least the portion of the modeled bioreactor is a first portion of the modeled bioreactor;
the process variables are first process variables;
the instructions further cause the one or more processors to receive a plurality of current values of second process variables, the second process variables describing second virtual contents of a second portion of the modeled bioreactor; and
computing the new values of the first process variables during the simulated time period is based in part on the received plurality of the current values of the second process variables.

Option 68, The non-transitory computer-readable medium of option 67, wherein computing the new values of the first process variables during the simulated time period includes computing a gradient of at least one of the first process variables based on a value of a corresponding one of the second process variables.

Option 69. The non-transitory computer-readable medium of any of the options 67-68, wherein the instructions further cause the one or more processors to determine one or more velocities associated with the virtual contents of the first portion of the modeled bioreactor.

Option 70. The non-transitory computer-readable medium of any of the options 67-69, wherein:
determining the one or more velocities is based at least in part on computational fluid dynamics.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept. Aspects are disclosed in the following clauses:

1. A method of computationally modeling a bioreactor, wherein the method comprises:

receiving a plurality of current values of process variables, the process variables describing virtual contents of at least a portion of the modeled bioreactor, and the virtual contents including virtual cellular biomass in a virtual

EP 4 617 960 A2

extracellular solution;

computing, by processing hardware of a computing system, new values of the process variables during a simulated time period, at least in part by

generating a plurality of constraints on flux rates of metabolic fluxes describing the virtual cellular biomass by modeling one or more effects of at least some of the current values of the process variables on metabolic reaction kinetics,

computing the flux rates of the metabolic fluxes by performing flux balance analysis subject to a metabolic objective and the generated plurality of the constraints on the flux rates,

computing rates of change of at least some of the process variables based at least in part on the computed flux rates, and

updating one or more of the current values of the process variables at least in part by integrating one or more of the computed rates of change for a virtual time step within the simulated time period;

computing, by the processing hardware, a metric of the computationally modeled bioreactor based at least in part on the computed new values of the process variables; and

generating, by the processing hardware, and based on the metric of the modeled bioreactor one or more of i) information displayed to a user via a user interface, ii) a control setting for a real-world bioreactor, or iii) a training set for an artificial intelligence model of a bioreactor.

2. The method of clause 1, wherein the process variables include at least one of: temperature, acidity or one or more variables indicative of total osmolarity of the virtual contents.

3. The method of clause 1, wherein the process variables include one or more variables indicative of concentration of the virtual cellular biomass and one or more variables indicative of extracellular metabolite concentrations in the virtual extracellular solution.

4. The method of clause 3, wherein the one or more variables indicative of the extracellular metabolite concentrations in the virtual extracellular solution include one or more variables indicative of concentrations of at least one of: oxygen, carbon dioxide, or ammonia.

5. The method of clause 3, wherein the one or more variables indicative of the extracellular metabolite concentrations in the virtual extracellular solution include variables indicative of concentrations of one or more of glucose, asparagine, glutamine, or glycine.

6. The method of clause 3, wherein the one or more variables indicative of the extracellular metabolite concentrations in the virtual extracellular solution include variables indicative of concentration of at least one target metabolic product.

7. The method of clause 3, wherein the process variables additionally include one or more variables indicative of one or more intracellular metabolite concentrations in the virtual cellular biomass.

8. The method of clause 1, wherein the plurality of the constraints on the flux rates includes an upper limit on at least one of i) glucose uptake, ii) glutamine uptake, iii) asparagine uptake, or iv) oxygen uptake.

9. The method of clause 1, wherein the plurality of the constraints on the rates of change includes a lower limit on an energy consumption rate for cellular maintenance.

10. The method of clause 1, wherein the one or more effects of at least some of the current values of the process variables on the metabolic reaction kinetics includes an effect of a value of at least one of:
i) temperature, ii) acidity, or iii) osmolarity on an upper limit of a metabolite uptake rate.

11. The method of clause 10,
wherein modeling the one or more effects of at least some of the current values of the process variables on the metabolic reaction kinetics includes computing the effect of the value of at least one of: i) temperature, ii) acidity, or iii) osmolarity on the upper limit of the metabolite uptake rate by multiplying the upper limit with a correction factor indicative of a reduction in the uptake rate due to non-ideal conditions.

12. The method of clause 1, wherein the one or more effects of the at least some of the current values of the process

32

variables on metabolic reaction kinetics includes an effect of stress on a lower limit of energy consumption rate for cellular maintenance.

13. The method of clause 12, wherein modeling the effect of stress includes computing the effect based at least **in** part on a cumulative effect of a temperature shift on the virtual cellular biomass.

14. The method of clause 1, wherein the one or more effects of the at least some of the current values of the process variables on metabolic reaction kinetics include an effect of concentration of at least one of metabolic byproducts.

15. The method of clause 1, wherein modeling the one or more effects includes calibrating the modeled effects with experimental data from a real-world cell culture.

16. The method of clause 1, wherein the metabolic objective includes minimizing a ratio of linear combinations of the at least some of the flux rates and squares of flux rates in the flux balance analysis.

17. The method of clause 1, wherein the metabolic objective includes minimizing a linear combination of at least some of the flux rates in the flux balance analysis.

18. The method of clause 17, wherein the linear combination is a sum of the fluxes in the flux balance analysis.

19. The method of clause 1, wherein the metabolic objective includes maximizing energy consumption rate for cellular maintenance.

20. The method of clause 1, wherein the metabolic objective includes maximizing a rate of growth of the virtual cellular biomass.

21. The method of clause 1, further comprising:

receiving one or more variables indicative of flow rates and compositions of one or more virtual input streams into the modeled bioreactor, and wherein
updating the at least some of the current values of the process variables includes accounting for the flow rates and the compositions of the one or more virtual input streams.

22. The method of clause 21, further comprising:

receiving one or more variables indicative of properties of a virtual output filter;
computing compositions of one or more virtual output streams from the modeled bioreactor using the properties of the virtual output filter, and wherein
updating the at least some of the current values of the process variables includes accounting for the compositions of the one or more virtual output streams.

23. The method of clause 21, wherein updating at least some of the current values of the process variables includes solving mass-balance equations.

24. The method of clause 1, further comprising:

receiving one or more control variables, and
updating an affected set of the current values of the process variables at least in part based on the received control variables.

25. The method of clause 1, wherein
the metric of the modeled bioreactor includes values of at least one of the process variables at different virtual times within the simulated time period.

26. The method of clause 1, wherein
the metric of the computationally modeled bioreactor indicates effectiveness or efficiency of the computationally modeled bioreactor in producing a target product.

27. The method of clause 1, wherein

the method comprises generating the control setting, and wherein
the method further comprises controlling an input to a real-world bioreactor based on the generated control setting.

28. The method of clause 1, wherein
the method comprises generating the training set for an artificial intelligence model of a bioreactor.

29. The method of clause 1, wherein
the virtual cellular biomass comprises virtual Chinese hamster ovary (CHO) cells.

30. The method of clause 1, wherein receiving the plurality of the current values includes at least one of

i) receiving values from a user via a graphical user interface;
ii) receiving values based on measurements of a real bioreactor;
iii) loading values from computer memory and based on previously computed values; or
iv) loading predetermined default values from computer storage.

31. The method of clause 1, wherein the modeled bioreactor is a spatially homogeneous bioreactor, and the at least the portion of the modeled bioreactor is an entirety of the spatially homogeneous bioreactor.

32. The method of clause 1, wherein:

the modeled bioreactor is a spatially heterogeneous bioreactor;
the at least the portion of the modeled bioreactor is a first portion of the modeled bioreactor;
the process variables are first process variables;
the method further includes receiving a plurality of current values of second process variables, the second process variables describing second virtual contents of a second portion of the modeled bioreactor; and
computing the new values of the first process variables during the simulated time period is based in part on the received plurality of the current values of the second process variables.

33. The method of clause 32, wherein computing the new values of the first process variables during the simulated time period includes computing a gradient of at least one of the first process variables based on a value of a corresponding one of the second process variables.

34. The method of clause 32, further comprising:
determining one or more velocities associated with the virtual contents of the first portion of the modeled bioreactor.

35. The method of clause 34, wherein:
determining the one or more velocities is based at least in part on computational fluid dynamics.

36. A non-transitory computer-readable medium storing instructions for computationally modeling a bioreactor, wherein the instructions, when executed by one or more processors, cause the one or more processors to:

receive a plurality of current values of process variables, the process variables describing virtual contents of at least a portion of the modeled bioreactor, and the virtual contents including virtual cellular biomass in a virtual extracellular solution;
compute, by processing hardware of a computing system, new values of the process variables during a simulated time period, at least in part by

generating a plurality of constraints on flux rates of metabolic fluxes describing the virtual cellular biomass by modeling one or more effects of at least some of the current values of the process variables on metabolic reaction kinetics,
computing the flux rates of the metabolic fluxes by performing flux balance analysis subject to a metabolic objective and the generated plurality of the constraints on the flux rates,
computing rates of change of at least some of the process variables based at least in part on the computed flux rates, and

updating one or more of the current values of the process variables at least in part by integrating one or more of the computed rates of change for a virtual time step within the simulated time period;

compute, by the processing hardware, a metric of the modeled bioreactor based at least in part on the computed new values of the process variables; and

generate, by the processing hardware, and based on the metric of the modeled bioreactor one or more of i) information displayed to a user via a user interface, ii) a control setting for a real-world bioreactor, or iii) a training set for an artificial intelligence model of a bioreactor.

37. The non-transitory computer-readable medium of clause 36, wherein the process variables include at least one of: temperature, acidity or one or more variables indicative of total osmolarity of the virtual contents.

38. The non-transitory computer-readable medium of clause 36, wherein the process variables include one or more variables indicative of concentration of the virtual cellular biomass and one or more variables indicative of extracellular metabolite concentrations in the virtual extracellular solution.

39. The non-transitory computer-readable medium of clause 38, wherein the one or more variables indicative of the extracellular metabolite concentrations in the virtual extracellular solution include one or more variables indicative of concentrations of at least one of: oxygen, carbon dioxide, or ammonia.

40. The non-transitory computer-readable medium of clause 38, wherein the one or more variables indicative of the extracellular metabolite concentrations in the virtual extracellular solution include variables indicative of concentrations of one or more of glucose, asparagine, glutamine, or glycine.

41. The non-transitory computer-readable medium of clause 38, wherein the one or more variables indicative of the extracellular metabolite concentrations in the virtual extracellular solution include variables indicative of concentration of at least one target metabolic product.

42. The non-transitory computer-readable medium of clause 38, wherein the process variables additionally include variables indicative of one or more intracellular metabolite concentrations in the virtual cellular biomass.

43. The non-transitory computer-readable medium of clause 36, wherein the plurality of the constraints on rates of change includes an upper limit on at least one of i) glucose uptake, ii) glutamine uptake, iii) asparagine uptake, or iv) oxygen uptake.

44. The non-transitory computer-readable medium of clause 36, wherein the plurality of the constraints on rates of change includes a lower limit on an energy consumption rate for cellular maintenance.

45. The non-transitory computer-readable medium of clause 36, wherein the one or more effects of at least some of the current values of the process variables on the metabolic reaction kinetics includes an effect of a value of at least one of: i) temperature, ii) acidity, or iii) osmolarity on an upper limit of a metabolite uptake rate.

46. The non-transitory computer-readable medium of clause 45, wherein
modeling the one or more effects of at least some of the current values of the process variables on the metabolic reaction kinetics includes computing the effect of the value of at least one of: i) temperature, ii) acidity, or iii) osmolarity on the upper limit of the metabolite uptake rate by multiplying the upper limit with a correction factor indicative of a reduction in the uptake rate due to non-ideal conditions.

47. The non-transitory computer-readable medium of clause 36, wherein the one or more effects of the at least some of the current values of the process variables on metabolic reaction kinetics include an effect of stress on a lower limit of energy consumption rate for cellular maintenance.

48. The non-transitory computer-readable medium of clause 47, wherein modeling the effect of stress includes computing the effect based at least in part on a cumulative effect of a temperature shift on the virtual cellular biomass.

49. The non-transitory computer-readable medium of clause 36, wherein the one or more effects of the at least some of the current values of the process variables on metabolic reaction kinetics include an effect of concentration of at least one of metabolic byproducts.

50. The non-transitory computer-readable medium of clause 36, wherein modeling the one or more effects include calibrating the modeled effects with experimental data from a real-world cell culture.

51. The non-transitory computer-readable medium of clause 36, wherein the metabolic objective includes minimizing a linear combination of at least some of the rates of change.

52. The non-transitory computer-readable medium of clause 36, wherein the metabolic objective includes minimizing a linear combination of at least some fluxes in the flux balance analysis.

53. The non-transitory computer-readable medium of clause 52, wherein the linear combination is a sum of the fluxes in the flux balance analysis.

54. The non-transitory computer-readable medium of clause 36, wherein the metabolic objective includes maximizing energy consumption rate for cellular maintenance.

55. The non-transitory computer-readable medium of clause 36, wherein the metabolic objective includes maximizing a rate of growth of the virtual cellular biomass.

56. The non-transitory computer-readable medium of clause 36, wherein the instructions further cause the one or more processors to:

receive one or more variables indicative of flow rates and compositions of one or more virtual input streams into the modeled bioreactor, and wherein
updating the at least some of the current values of the process variables includes accounting for the flow rates and the compositions of the one or more virtual input streams.

57. The non-transitory computer-readable medium of clause 56, wherein the instructions further cause the one or more processors to:

receive one or more variables indicative of properties of a virtual output filter;
compute compositions of one or more virtual output streams from the modeled bioreactor using the properties of the virtual output filter, and wherein
updating the at least some of the current values of the process variables includes accounting for the compositions of the one or more virtual output streams.

58. The non-transitory computer-readable medium of clause 56, wherein updating the at least some of the current values of the process variables includes solving mass-balance equations.

59. The non-transitory computer-readable medium of clause 36, wherein the instructions further cause the one or more processors to:

receive one or more control variables, and
update an affected set of the current values of the process variables at least in part based on the received control variables.

60. The non-transitory computer-readable medium of clause 36, wherein
the metric of the modeled bioreactor includes values of at least one of the process variables at different virtual times within the simulated time period.

61. The non-transitory computer-readable medium of clause 36, wherein
the metric of the modeled bioreactor indicates effectiveness or efficiency of the modeled bioreactor in producing a target product.

62. The non-transitory computer-readable medium of clause 36, wherein the instructions

cause the one or more processors to generate the control setting, and
further cause the one or more processors to control an input to a real-world bioreactor based on the generated control setting.

63. The non-transitory computer-readable medium of clause 36, wherein the instructions cause the one or more processors to generate the training set for an artificial intelligence model of a bioreactor.

64. The non-transitory computer-readable medium of clause 36, wherein the virtual cellular biomass comprises virtual Chinese hamster ovary (CHO) cells.

65. The non-transitory computer-readable medium of clause 36, wherein receiving the plurality of the current values includes at least one of:

   i) receiving values from a user via a graphical user interface;
   ii) receiving values based on measurements of a real bioreactor;
   iii) loading values from computer memory and based on previously computed values; or
   iv) loading predetermined default values from computer storage.

66. The non-transitory computer-readable medium of clause 36, wherein the modeled bioreactor is a spatially homogeneous bioreactor, and the at least the portion of the modeled bioreactor is an entirety of the spatially homogeneous bioreactor.

67. The non-transitory computer-readable medium of clause 36, wherein:

   the modeled bioreactor is a spatially heterogeneous bioreactor;
   the at least the portion of the modeled bioreactor is a first portion of the modeled bioreactor;
   the process variables are first process variables;
   the instructions further cause the one or more processors to receive a plurality of current values of second process variables, the second process variables describing second virtual contents of a second portion of the modeled bioreactor; and
   computing the new values of the first process variables during the simulated time period is based in part on the received plurality of the current values of the second process variables.

68. The non-transitory computer-readable medium of clause 67, wherein computing the new values of the first process variables during the simulated time period includes computing a gradient of at least one of the first process variables based on a value of a corresponding one of the second process variables.

69. The non-transitory computer-readable medium of clause 67, wherein the instructions further cause the one or more processors to determine one or more velocities associated with the virtual contents of the first portion of the modeled bioreactor.

70. The non-transitory computer-readable medium of clause 69, wherein:
determining the one or more velocities is based at least in part on computational fluid dynamics.

**Claims**

1. A method of computationally modeling a bioreactor, wherein the method comprises:

   receiving a plurality of current values of process variables, the process variables describing virtual contents of at least a portion of the modeled bioreactor, and the virtual contents including virtual cellular biomass in a virtual extracellular solution;
   computing, by processing hardware of a computing system, new values of the process variables during a simulated time period, at least in part by

      generating a plurality of constraints on flux rates of metabolic fluxes describing the virtual cellular biomass by modeling one or more effects of at least some of the current values of the process variables on metabolic reaction kinetics,
      computing the flux rates of the metabolic fluxes by performing flux balance analysis subject to a metabolic objective and the generated plurality of the constraints on the flux rates,
      computing rates of change of at least some of the process variables based at least in part on the computed flux rates, and

updating one or more of the current values of the process variables at least in part by integrating one or more of the computed rates of change for a virtual time step within the simulated time period;

computing, by the processing hardware, a metric of the computationally modeled bioreactor based at least in part on the computed new values of the process variables; and

generating, by the processing hardware, and based on the metric of the modeled bioreactor one or more of i) information displayed to a user via a user interface, ii) a control setting for a real-world bioreactor, or iii) a training set for an artificial intelligence model of a bioreactor.

2. The method of claim 1, wherein the process variables include:

1) at least one of: temperature, acidity or one or more variables indicative of total osmolarity of the virtual contents, or

2) one or more variables indicative of concentration of the virtual cellular biomass and one or more variables indicative of extracellular metabolite concentrations in the virtual extracellular solution, optionally wherein the one or more variables indicative of the extracellular metabolite concentrations in the virtual extracellular solution include one or more variables indicative of concentrations of at least one of: oxygen, carbon dioxide, ammonia, glucose, asparagine, glutamine, glycine or at least one target metabolic product, optionally wherein the process variables additionally include one or more variables indicative of one or more intracellular metabolite concentrations in the virtual cellular biomass.

3. The method of claim 1, wherein the plurality of the constraints on the flux rates includes an upper limit on at least one of i) glucose uptake, ii) glutamine uptake, iii) asparagine uptake, or iv) oxygen uptake or the plurality of the constraints on the rates of change includes a lower limit on an energy consumption rate for cellular maintenance.

4. The method of claim 1, wherein the one or more effects of at least some of the current values of the process variables on the metabolic reaction kinetics includes:

1) an effect of a value of at least one of i) temperature, ii) acidity, or iii) osmolarity on an upper limit of a metabolite uptake rate, optionally wherein modeling the one or more effects of at least some of the current values of the process variables on the metabolic reaction kinetics includes computing the effect of the value of at least one of: i) temperature, ii) acidity, or iii) osmolarity on the upper limit of the metabolite uptake rate by multiplying the upper limit with a correction factor indicative of a reduction in the uptake rate due to non-ideal conditions;

2) an effect of stress on a lower limit of energy consumption rate for cellular maintenance, optionally wherein modeling the effect of stress includes computing the effect based at least in part on a cumulative effect of a temperature shift on the virtual cellular biomass; or

3) an effect of concentration of at least one of metabolic byproducts.

5. The method of claim 1, wherein modeling the one or more effects includes calibrating the modeled effects with experimental data from a real-world cell culture.

6. The method of claim 1, wherein the metabolic objective includes:

1) minimizing a ratio of linear combinations of the at least some of the flux rates and squares of flux rates in the flux balance analysis; or

2) minimizing a linear combination of at least some of the flux rates in the flux balance analysis, optionally wherein the linear combination is a sum of the fluxes in the flux balance analysis.

7. The method of claim 1, wherein the metabolic objective includes maximizing energy consumption rate for cellular maintenance or maximizing a rate of growth of the virtual cellular biomass.

8. The method of claim 1, further comprising:

1) receiving one or more variables indicative of flow rates and compositions of one or more virtual input streams into the modeled bioreactor, and wherein updating the at least some of the current values of the process variables includes accounting for the flow rates and the compositions of the one or more virtual input streams, optionally wherein updating at least some of the current values of the process variables includes solving mass-balance equations, optionally further comprising:

receiving one or more variables indicative of properties of a virtual output filter;

computing compositions of one or more virtual output streams from the modeled bioreactor using the properties of the virtual output filter, and wherein updating the at least some of the current values of the process variables includes accounting for the compositions of the one or more virtual output streams; or

2) receiving one or more control variables, and updating an affected set of the current values of the process variables at least in part based on the received control variables.

9. The method of claim 1, wherein

1) the metric of the modeled bioreactor includes values of at least one of the process variables at different virtual times within the simulated time period; or

2) the metric of the computationally modeled bioreactor indicates effectiveness or efficiency of the computationally modeled bioreactor in producing a target product.

10. The method of claim 1, wherein

1) the method comprises generating the control setting, and wherein the method further comprises controlling an input to a real-world bioreactor based on the generated control setting, or

2) the method comprises generating the training set for an artificial intelligence model of a bioreactor.

11. The method of claim 1, wherein receiving the plurality of the current values includes at least one of

i) receiving values from a user via a graphical user interface;

ii) receiving values based on measurements of a real bioreactor;

iii) loading values from computer memory and based on previously computed values; or

iv) loading predetermined default values from computer storage.

12. The method of claim 1, wherein:

the modeled bioreactor is a spatially heterogeneous bioreactor;

the at least the portion of the modeled bioreactor is a first portion of the modeled bioreactor;

the process variables are first process variables;

the method further includes receiving a plurality of current values of second process variables, the second process variables describing second virtual contents of a second portion of the modeled bioreactor; and

computing the new values of the first process variables during the simulated time period is based in part on the received plurality of the current values of the second process variables.

13. The method of claim 12, wherein computing the new values of the first process variables during the simulated time period includes computing a gradient of at least one of the first process variables based on a value of a corresponding one of the second process variables.

14. The method of claim 12, further comprising:

determining one or more velocities associated with the virtual contents of the first portion of the modeled bioreactor, optionally wherein determining the one or more velocities is based at least in part on computational fluid dynamics.

15. A non-transitory computer-readable medium storing instructions that, when executed by one or more processors, cause the one or more processors to perform the method of any one of claims 1-14.

**FIG. 1**

200

FIG. 2

300

FIG. 3

400

410

## Experimental Conditions

### Process Conditions

422a — Working Volume (L)
[ ] [ UPDATE ] 424a

422b — Seed Density ($10^5$ cells/mL)
[ ] [ UPDATE ] 424b

422c — pH
[ ] [ UPDATE ] 424c

422d — Filter Change (Days)
[ ] [ UPDATE ] 424d

422e — Filter Types
[ ] [ UPDATE ] 424e

422f — Harvest Time (Days)
[ ] [ UPDATE ] 424f

### Process Conditions

422g — Initial Temperature (°C)
[ ] [ UPDATE ] 424g

422h — Production Temperature (°C)
[ ] [ UPDATE ] 424h

422i — Temperature Shift Time (Days)
[ ] [ UPDATE ] 424i

422j — Dilution Rates (per Day)
[ ] [ UPDATE ] 424j

422k — Dilution Times (Days)
[ ] [ UPDATE ] 424k

422l — Glucose Shots Flow (mL/hr)
[ ] [ UPDATE ] 424l

422m — Glucose Shots Times (Days)
[ ] [ UPDATE ] 424m

**FIG. 4A**

400

412

## Media Composition

**Batch Media**

432a — Glucose Concentration (mM)
[          ] [ UPDATE ]
434a

432b — Glutamine Concentration (mM)
[          ] [ UPDATE ]
434b

432c — Glutamate Concentration (mM)
[          ] [ UPDATE ]
434c

432d — Cystine Concentration (mM)
[          ] [ UPDATE ]
434d

432e — Glycine Concentration (mM)
[          ] [ UPDATE ]
434e

432f — Serine Concentration (mM)
[          ] [ UPDATE ]
434f

432g — Aspartate Concentration (mM)
[          ] [ UPDATE ]
434g

432h — Asparagine Concentration (mM)
[          ] [ UPDATE ]
434h

**Perfusion Media**

442a — Glucose Concentration (mM)
[          ] [ UPDATE ]
444a

442b — Glutamine Concentration (mM)
[          ] [ UPDATE ]
444b

442c — Glutamate Concentration (mM)
[          ] [ UPDATE ]
444c

442d — Cystine Concentration (mM)
[          ] [ UPDATE ]
444d

442e — Glycine Concentration (mM)
[          ] [ UPDATE ]
444e

442f — Serine Concentration (mM)
[          ] [ UPDATE ]
444f

442g — Aspartate Concentration (mM)
[          ] [ UPDATE ]
444g

442h — Asparagine Concentration (mM)
[          ] [ UPDATE ]
444h

**FIG. 4B**

500

**Process Variables**

Biomass

514a
512a
510a

Lactate

512b
514b
510b

Glucose

512c
514c
510c

NH3

514d
512d
510d

PERMEATE

514e
512e
510e

ANTI

514f
512f
510f

**FIG. 5**

600

620

610

Computer

630

Controller

640

Sensor(s)

Bioreactor

**FIG. 6**

EP 4 617 960 A2

700

Receive a plurality of current values of process variables — 710

Generate constraints on flux rates of metabolic fluxes by modeling one or more effects of at least some of the current values of the process variables on metabolic reaction kinetics — 720

Compute the flux rates of the metabolic fluxes by performing flux balance analysis subject to a metabolic objective and the generated plurality of the constraints on the flux rates — 730

Compute the rates of change of the at least some of the process variables based at least in part on the computed flux rates — 740

Update one or more of the current values of the process variables by integrating the computed rates of change for a virtual time step — 750

Compute a metric of the computationally modeled bioreactor — 760

Generate information displayed to a user via a user interface, a control setting for a bioreactor, and/or a training set for an artificial intelligence model. — 770

**FIG. 7**

45

FIG. 8

FIG. 9B

FIG. 9A